(19) Europäisches Patentamt
European Patent Office
Office européen des brevets

(11) Publication number: **0 620 010 A1**

(12) **EUROPEAN PATENT APPLICATION**

(21) Application number: **94106294.5**

(22) Date of filing: **09.10.90**

(51) Int. Cl.5: **A61K 39/21**, C07K 7/10, G01N 33/569

This application was filed on 22 - 04 - 1994 as a divisional application to the application mentioned under INID code 60.

(30) Priority: **12.10.89 US 420469**

(43) Date of publication of application: **19.10.94 Bulletin 94/42**

(60) Publication number of the earlier application in accordance with Art.76 EPC: **0 426 314**

(84) Designated Contracting States: **AT BE CH DE DK ES FR GB GR IT LI LU NL SE**

(71) Applicant: **VIRAL TECHNOLOGIES, INC.**
**Two Democracy Center**
**6903 Rockledge Drive**
**Suite 1200**
**Bethesda, Maryland 20817 (US)**

Applicant: **THE GEORGE WASHINGTON UNIVERSITY**
**Office of Sponsored Research,**
**Rice Hall,**
**Room 601,**
**2121 Eye Street, N.W.**
**Washington, D.C. 20052 (US)**

(72) Inventor: **Naylor, Paul H.**
**4003 Woodhaven Lane**
**Bowie, Maryland 20715 (US)**
Inventor: **Wang, Su Sun**
**1512 Chula Vista Drive**
**Belmont, California 94002 (US)**

(74) Representative: **Wise, Stephen James et al**
**c/o RAWORTH, MOSS & COOK**
**36 Sydenham Road**
**Croydon, Surrey CR0 2EF (GB)**

(54) **HIV related peptides.**

(57) Fragments of HIV p17 protein ranging in length from about 12 to about 40 amino acids are used to form diagnostics and vaccines for detection or treatment of AIDS. Specific peptide fragments extend from the N-terminal to the C-terminal. Peptides having the amino acid sequences shown by the following formula (I), (II), (III), (IV) and (V):

EP 0 620 010 A1

Tyr-Ser-Val-His-Gln-Arg-Ile-Asp-Val-Lys-
Asp-Thr-Lys-Glu-Ala-Leu-Gly-Lys-Ile-Glu-
Glu-Glu-Gln-Asn-Lys-Ser-Lys-Lys-Lys-Ala (I)

Gly-Ala-Arg-Ala-Ser-Val-Leu-Ser-Gly-Gly-
Glu-Leu-Asp-Arg-Trp-Glu-Lys-Ile-Arg-Leu-
Arg-Pro-Gly-Gly-Lys-Lys-Lys-Tyr-Lys-Leu-
Lys-His (II)

Ile-Val-Trp-Ala-Ser-Arg-Glu-Leu-Glu-Arg-
Phe-Ala-Val-Asn-Pro-Gly-Leu-Leu (III)

Glu-Thr-Ser-Glu-Gly-Cys-Arg-Gln-Ile-Leu-
Gly-Gln-Leu-Gln-Pro-Ser-Leu-Gln-Thr-Gly-
Ser-Glu-Glu-Leu-Arg-Ser-Leu-Tyr-Asn-Thr-
Val-Ala-Thr-Leu (IV)

Ala-Gln-Gln-Ala-Ala-Ala-Asp-Thr-Gly-His-
Ser-Ser-Gln-Val-Ser-Gln-Asn-Tyr (V)

are immunoreactive to antibodies in sera of patients testing seropositive for p17 of HIV-1.

FIG. 1

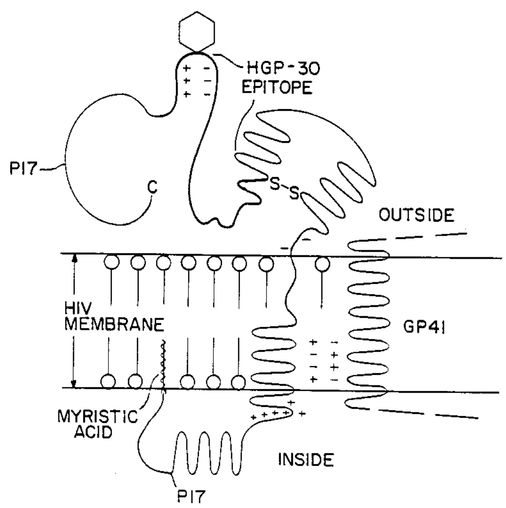

## BACKGROUND OF THE INVENTION

### (1) Field Of The Invention

This invention relates to peptide fragments derived from p17 gag protein of human immunodeficiency virus (HIV) which is the causative organism of the diseases known as Acquired Immune Deficiency Syndrome (AIDS) or pre-AIDS (also known as AIDS-Related Complex or ARC) . These peptide fragments can be used directly as diagnostic reagents for detecting the presence in the blood of the HIV retrovirus, or for detecting antibodies to HIV retroviral proteins or for raising antibodies which recognize the p17 gag protein of HIV or its fragments. The invention also relates to fragments or analogues of the p17 gag protein peptides which possess the immunogenic epitopes of the "parent" peptides. In a further aspect, the invention relates to the use of these p17 peptide fragments and analogues which are capable of eliciting protective antibodies as a component of an AIDS vaccine and to the resulting AIDS vaccine compositions.

### (2) Discussion of the Prior Art

There has been extensive amounts of research over the past several years, first to identify the cause of the disease AIDS and after the positive identification of the retroviruses HTLV-III, ARV and others, now generically referred to as HIV, as the causative organism, efforts have concentrated on more detailed analysis of the genetic makeup, molecular biology, infection mechanism, biochemistry, high sensitivity methods of detection and treatments, therapies and cures. Extensive progress has been made in all of these areas yet much more work needs to be done to effectively combat the spread of AIDS. An essential part of the approach for combatting the spread of the highly infectious HIV virus is the development of highly sensitive tests for diagnosing the presence in the blood of the HIV virus or antibodies to the HIV virus. Early detection may facilitate therapies for combatting or slowing down the progression of the course of HIV infection leading to the development of full-blown or frank AIDS. Knowledge of infection with HIV by accurate diagnosis may encourage infected patients to alter or modify their lifestyles in such manner as to reduce the risk of spreading the virus. Additionally, identification of protective antibodies based on epitope recognition can offer a more effective mechanism for staging and/or diagnosing the AIDS or pre-AIDS/ disease. Such staging and early diagnosis of seropositive individuals may then allow for vaccinations to provide the appropriate protective antibodies for treating the seropositive individual.

Recently, U.S. Patent 4,520,113 to Gallo, et al. described a method of detecting antibodies in sera of AIDS and pre-AIDS patients by using lysates of a cell line, designated H9/HTLV-III as the antigen in an enzyme-linked immunosorbent assay (ELISA), in a strip radioimmunoassay (RIA) based on the Western Blot technique, or in an indirect immunofluorescent method. The major immune reactivity or specificity was directed against p41(MW 41,000), a protein constituting the envelope antigen of HIV. Other antigens including p65(MW 65,000), p60(MW 60,000), p55(MW 55,000), and p24(MW 24,000) were also recognized by antibodies in the tested sera. This method does not have 100% accuracy.

U.S. Patent 4,735,896 to C. Y. Wang and J. G. Wang provide a method for detection and diagnosis of AIDS, ARC and pre-AIDS conditions in body fluid by use of a chemically synthesized peptide having a sequence of about 21 amino acids corresponding to a segment of the transmembrane protein p41 of the HTLV-III strain of HIV.

However, the envelope proteins of the various strains of HIV are subject to genetic drift, i.e. variations of the amino acid sequences, such that, antibodies to one envelope protein might not bind to a virus with even a slightly different envelope protein.

In commonly assigned, copending application Serial No. 864,599 filed May 19, 1986 to A. Goldstein and S. Wang (corresponding to published European Application 0,246,829, published November 25, 1987) a different approach to diagnosis and treatment of AIDS, ARC and pre-AIDS, as well as an AIDS vaccine was proposed. This approach was based, in part, upon the observation, confirmed by other researchers, that the group antigen specific protein p17 (also referred to by some researchers as p18) includes a more highly conserved region. Unlike the shifting envelope or surface proteins of HIV, the overall amino acid sequencing of p17 (gag) protein is essentially the same in all strains of HIV found to date. Furthermore, recent investigations have discovered that antibodies to p17 proteins are present in healthy HIV-infected individuals and decline as they progress to frank AIDS. The presence of p17 antibodies in human serum is also required for effective antibody-dependent cellular immunity against viral infected cells. See e.g. J.M.A. Lange, et al. "Decline of Antibody Reactivity to Outer Viral Core protein p17 is an Earlier Serological Marker of Disease Progression in Human Immunodeficiency Virus Infection than Anti-p24 Decline," AIDS, vol. 1, No. 3, 155-159 (1987). The reference in the title to p17 as "outer viral core protein" is based on the recent

observations by H. Gelderblom and others that the p17 protein, unlike the p24 inner core protein, is localized at or near the surface of the virion structure (H. Gelderblom, et al. "Fine Structure of Human Immunodeficiency Virus (HIV) and Immunolocalization of Structural Protein," Virology, Vol. 156, 171-176 (1987)). These recent observations concerning the localization of the p17 protein on the outer surface coupled with the association of decline of p17 antibodies with progression of disease lends credence to the Goldstein and Wang approach to developing an AIDS vaccine and diagnostic which is based on synthetic peptides comprised of an amino acid sequence incorporating the 18 amino acids sequence at positions 92 to 109 of the p17 gag protein of HIV. More specifically, the 30-mer [Ser-86] peptide, designated HGP-30, between positions 85 and 114[1] of HIV-1 strain SF-2, and having the amino acid sequence of formula (I):

Tyr-Ser-Val-His-Gln-Arg-Ile-Asp-Val-Lys-
Asp-Thr-Lys-Glu-Ala-Leu-Glu-Lys-Ile-Glu-
Glu-Glu-Gln-Asn-Lys-Ser-Lys-Lys-Lys-Ala (I)

has been shown to elicit antibodies effective in neutralizing various different strains of HIV in in vitro studies, i.e. is group specific rather than strain specific. In fact, based on the successful safety and immunogenicity studies on rabbits, monkeys and chimpanzees, clinical trials to test the in vivo safety and effectiveness of HGP-30 based vaccine have been initiated.

International PCT application WO 86/01827, published March 27, 1986 by Alizon, et al. of Institute Pasteur and Centre National de 1a Recherche Scientifique is particularly directed to DNA sequences or fragments thereof which are hybridizable with tie genomic RNA of the LAV virus including the DNA fragments which encode for each of the intact envelope and core proteins. While no specific examples are given, it is broadly suggested that the utility of the DNA include expression of the viral antigens for diagnostic purposes as well as for the production of a vaccine.

European Patent Application 0,230,222 A1, published July 29, 1907, is directed to a polypeptide immunologically equivalent to the gag-protein products of HTLV-III having the amino acid sequence given in either Figure 8 or Figure 16 or the 14Kd, 16Kd or 24Kd proteolytic polypeptide fragments thereof or polypeptides related to any of these polypeptides by amino acid substitutions which occur through mutations of a recombinant host cell which produces the polypeptides. This publication is also concerned with the genes and recombinant expression vectors, transformed cells, processes for producing polypeptides using the transformed host cells, method for testing human blood, vaccines and test kits. The examples show the production of expression plasmids for gag-protein fragments and growth of yeast cultures containing the gag expression plasmids as well as purification, polyacrylamide gel electrophoresis and Western Blot analysis, cell labeling and a general description of a diagnostic test for AIDS.

U.K. Patent Application G.B. 2,181,435A, published April 23, 1987, to Hu, et al. (Oncogen) includes challenge data in chimpanzees but only with respect to envelope protein products. It is suggested, however, that the gag related proteins produced by recombinant vaccinia viruses are immunoreactive proteins containing the major epitopes of the authentic core proteins. However, the major epitopes are not defined. On page 12, lines 34-44 it is suggested that any restriction enzyme may be used to generate fragments containing the envelope or gag sequences provided the enzymes do not destroy the antigenicity of protein gene product, the antigenic site consisting of about 7 to about 14 amino acids. With regard to the formulation of a vaccine, it is suggested that the vaccines include those containing envelope relating epitopes formulated alone or in combination with other epitopes such as the gag related epitopes. Reference is made to early studies that AIDS patients in early symptom-free stages makes antibodies against both envelope and core proteins but as the disease progresses and symptoms appear, the anti-gag antibodies are reduced. Therefore, the inclusion of gag-related epitopes in vaccine formulations in im-

[1] In the present application, the amino acid number sequence has been modified from the numbering sequence used in prior application Serial No. 864,599 wherein HGP-30 corresponds to the amino acid sequence between positions 86 and 115; this difference is based on the different numbering systems used by geneticists (see e.g. Human Retroviruses and AIDS, 1987 Ed. by G. Myers, et al., published by Theoretical Biology and Biophysics Groups, Los Alamos National Laboratory, Los Alamos, New Mexico 87545) and protein chemists, and particularly with regard to position 1 at the N-terminal position. Geneticists begin the number of the p17 gag peptide of HIV 1 with methionine (Met) at position 1 whereas in the present invention position 1 is considered as the myristylated glycine (Gly) N-terminal.

munoprophylaxis or immunotherapy of LAV of HTLV-III related disease is suggested. Nevertheless, there are no direct suggestions that the gag-proteins, per se, or their antibodies would, by themselves, provide useful vaccines. Various sections relate to Baculovirus gag recombinants, vaccinia gag recombinants, and to the expression of gag related protein tissue culture cells. It is concluded that the expression products were immunoreactive to monoclonal antibodies against p25 and p18.

None of the above three published patents identify any specific p17 (or p15) peptides or peptide fragments.

More recently, several independent groups of researchers have been able to generate neutralizing monoclonal antibodies to HIV p17, although monoclonals to p24, p41 and different epitopes of p17 were not effective. The monoclonal antibodies to p17 which were effective in the various neutralization assays bound to HGP-30. Conversely, the neutralizing anti-envelope antibody did not bind to HGP-30. See, for example, Papsidero, L. D., et al., "Human Immunodeficiency Virus Type 1-Neutralizing Monoclonal Antibodies Which React With p17 Core Protein's Characterization and Epitope Mapping," J. Virology, Vol. 63, No. 1, 267-272 (1989); Yoshihara, P., et al., "HIV Neutralization Studies Using Anti-Core and Anti-Envelope Protein Monoclonal Antibodies," IV Int. Conf. on AIDS, Abstract #3071. Representative data is shown in the following Table 1:

Table 1

| Neutralizing Monoclonal Antibodies to HIV p17 Recognize HGP-30 | | | | |
|---|---|---|---|---|
| Source | Neutralization | Western Blot | HIV | HGP-30 |
| Sarin | + | p17/p55 | 1600 | 1600 |
| Yoshihara | + | p17/p55 | 1600 | 400 |
| Ruscetti | + | p17/p55 | 200 | 800 |
| Ruscetti | + | p17/p55 | 100 | 200 |
| Ruscetti | + | gp 160 | 800 | 0 |
| Yoshihara | - | p24/p55 | 1600 | 0 |
| Yoshihara | - | 0 | 0 | 0 |

Source indicates the scientist responsible for the antibody and neutralization data.

Titers represent dilution which is at least twice the control value in ELISA's using either whole HIV extract or synthetic HGP-30 peptide.

Nevertheless, there have been only a few studies describing purification of large amounts of p17 and even fewer attempts to define immunogenic epitopes on native p17 in cases of natural infection. There have been "unpublished" studies which show a lack of neutralizing activity of whole p17, however, these studies may be flawed by presence of immunodominant but non-neutralizing epitopes. Other studies were flawed as a result of using too small epitopes, e.g. less than 10 amino acids in length, which may result in inappropriate epitope folding and presentation.

It is probable however, that not all epitopes on p17 will generate neutralizing antibodies; a conclusion which is consistent with the data showing that not all monoclonal antibodies to p17 are effective to neutralize in vitro (cf. Yoshihara, et al., ibid). Such epitopes may, however, generate protective antibodies which function through mechanisms other than inhibition of in vitro virus infection. These mechanisms might include antibody dependent cellular mediated cytotoxicity or T-cell mediated cytotoxicity.

Accordingly, it is an object of this invention to provide peptides which exhibit epitopes of natural p17 gag HIV protein and which are immunoreactive with antibodies in patients with AIDS, pre-AIDS and ARC conditions. Such peptides would, by definition, be candidates for diagnostics and vaccines.

It is another object of this invention to provide diagnostic reagents which are effective to accurately measure the presence and levels of p17 antibodies which recognize specific epitopes of p17 in the body fluids of patients exposed to HIV.

A further related object of the invention is to provide a diagnostic method and kit capable of making a serological determination of the presence or absence of the AIDS virus particles containing p17 epitopes defined by the peptides.

Still another object of this invention is to provide novel immunogens by bonding any of the novel peptides to each other or to an immunogenic carrier material and to the use of these immunogens for raising antibodies to specific epitopes of p17 which, in turn, could be used in immunoassay formats to

measure p17 or fragments thereof in culture fluids or body fluids.

Another object of this invention is to provide immunogenic materials capable of raising antibodies which are effective in neutralizing HIV and, therefore, as active ingredients in an AIDS vaccine.

It is still another object of this invention to provide specific peptides to eliminate harmful or unuseful antibodies from serum or to enrich for useful antibodies in serum and to use such processed serum in an immunoprophylactic setting to protect infected individuals who are not able to raise appropriate protective antibodies in a vaccination setting.

## BRIEF DESCRIPTION OF THE DRAWINGS

Figure 1 is a schematic drawing illustrating the proposed orientation for the p17 gag protein of HIV;
Figure 2 is a chart depicting the primary amino acid sequences of HIV-1 p17 (strain HB10) and also of the p17 proteins of HIV-2 and SIV;
Figure 3 is another chart depicting the primary amino acid sequence of the peptides in p17;
Figure 4 is a graph plotting antibody titers of HIV p17 peptides in rabbits immunized with native HIV p17; and
Figure 5 is a bar graph illustrating the results of ELISA analysis of sera from 4 Rhesus monkeys exposed to live HIV antibodies to the p17 peptide fragments of this invention.

## SUMMARY OF THE INVENTION

According to the invention, it has been found that the p17 gag HIV protein can be divided into five major segments or fragments, each possessing potentially important immunogenic epitopes, these five peptides being designated, starting from the N-terminal end: Peptide A (1-32), Peptide B (32-50), Peptide C (51-84), Peptide D (85-114) and Peptide E (114-131); wherein Peptide D is HGP-30 which is the 30-mer peptide disclosed in copending application Serial No. 864,599, the entire disclosure of which is incorporated herein by reference thereto. Each of these peptides possess unique structural features but have in common the presence of epitopes which are recognized by antibodies in various individuals who are seropositive for HIV p17. Accordingly, each of these peptides provide useful reagents for the diagnosis of the body fluids of individuals to determine the presence of antibodies to HIV p17 and, for individuals exposed to HIV, to determine the course of progression of the infection as a measure of antibodies to p17 found in the blood or other body fluids.

More specifically, the above and other objects of the invention which will become apparent by reference to the following detailed discussion and preferred embodiments and with the aid of the accompanying drawings, are accomplished, in accordance with one aspect thereof, by one or more synthetic peptides, presenting common immunogenic epitopes with p17 gag protein of human immunodeficiency virus, HIV, and especially HIV-1, said peptide(s) being selected from peptides having the amino acid sequences corresponding to a part or the entirety of the amino acid sequences of the following formulas (II), (III), (IV) and (V), including the analogues thereof, wherein one or more of the amino acids in the sequence may be substituted, deleted or added, so long as the immunogenic reactivity to antibodies to HIV p17 derived from the three-dimensional conformation of the sequence is preserved, and also including conjugates of the peptides or analogues thereof:

Peptide A (Positions 1-32)

Gly-Ala-Arg-Ala-Ser-Val-Leu-Ser-Gly-Gly-
Glu-Leu-Asp-Arg-Trp-Glu-Lys-Ile-Arg-Leu-
Arg-Pro-Gly-Gly-Lys-Lys-Lys-Tyr-Lys-Leu-
Lys-His (II)

Peptide B (Positions 33-50)

Ile-Val-Trp-Ala-Ser-Arg-Glu-Leu-Glu-Arg-
Phe-Ala-Val-Asn-Pro-Gly-Leu-Leu (III)

Peptide C (Positions 51-84)

Glu-Thr-Ser-Glu-Gly-Cys-Arg-Gln-Ile-Leu-
Gly-Gln-Leu-Gln-Pro-Ser-Leu-Gln-Thr-Gly-
Ser-Glu-Glu-Leu-Arg-Ser-Leu-Tyr-Asn-Thr-
Val-Ala-Thr-Leu (IV)

Peptide E (Positions 114-131)

Ala-Gln-Gln-Ala-Ala-Ala-Asp-Thr-Gly-
His-Ser-Ser-Gln-Val-Ser-Gln-Asn-Tyr (V)

wherein the peptide having the amino acid sequence of formula (II) contains from about 26 to about 40 amino acids, the peptide having the amino acid sequence of formula (III) contains from about 14 to about 27 amino acids, the peptide having the amino acid sequence of formula (IV) contains from about 28 to about 42 amino acids, and the peptide of formula (V) contains from about 12 to about 25 amino acids.

According to another aspect of the invention there is provided a composition which is a mixture of a peptide having specific immuno-reactivity with antibodies to HIV p17 selected from the group consisting of peptide containing from about 10 to about 36 amino acids, the sequence of which corresponds to part or the entirety of the sequence of formula (I') (including Peptide D)

(Positions 91-108)

Ile-$X_1$-$X_2$-Lys-Asp-Thr-Lys-Glu-Ala-Leu-$X_3$-
Lys-Ile-Glu-Glu-Glu-Gln-Asn (I')

wherein $X_1$ is Glu or Asp, $X_2$ is Ile or Val and $X_3$ is Asp or Glu, and conjugates and analogues thereof;

and at least one of the peptides of formula (II), (III), (IV) and (V).

In still another aspect of the invention there is provided a peptide having from 10 to 14 amino acids including the following sequence of formula (I'')

Ile-$X_1$-$X_2$-Lys-Asp-Thr-Lys-Glu-Ala-Leu (I'')

wherein $X_1$ is Glu or Asp, and $X_2$ is Ile or Val, the peptide having specific immunoreactivity with antibodies

to HIV p17.

According to still another feature of the invention any of the foregoing peptides is covalently bonded to an immunogenic carrier material to form an immunogenic antigen. Preferably, when used in a diagnostic kit, the peptide is labelled for serological detection of the antigen by an antibody to the antigen.

The invention also provides, in another aspect thereof, a vaccine for use in the prevention or treatment of Acquired Immune Deficiency Syndrome (AIDS) or AIDS-Related Complex (ARC) which includes a therapeutically effective amount of one or more of the above-described peptides of formula (II), (III), (IV) or (V) or a mixture thereof with the peptide of formula (I').

There is also provided by this invention a method for decreasing viremia associated with AIDS which involves administering to an individual exposed to the AIDS virus, at risk for AIDS, or infected with AIDS, a therapeutically effective amount of at least one of the antigenic peptides of formula (II), (III), (IV) or (V) or a mixture thereof with a peptide of formula (I').

A still further aspect of the invention is a method for detection of the AIDS virus in body fluids of a human patient. According to this method one or more samples of the body fluid to be tested is contacted with one or more of the peptides of formula (II), (III), (IV) or (V) or a mixture thereof with a peptide of formula (I'), and thereafter measuring the formation of an antigen-antibody complex, for example, by radioimmunoassay, enzyme linked immunosorbent assay, or indirect immunofluorescent assay. When a mixture of peptides is used, they may be used in a single assay in which case the formation of antigen-antibody complex for each peptide in the mixture is measured, or they may be used in separate assays for different samples of body fluid. In the latter case, the formation of antigen-antibody complex for each sample is measured.

As one means for carrying out the foregoing diagnostic method for detection of the AIDS virus the invention also provides a diagnostic test kit for detection of antibodies recognizing epitopes of p17 protein of HIV in human body fluid. The kit includes a compartmented enclosure containing multiwell plates which are coated with one or more of the p17 peptide fragments or analogs according to the invention, and ELISA materials for enzyme detection including normal animal antisera and enzyme, labelled anti-antibody sera and a color change indicator. The wells of each plate may be coated with different ones of the peptides. In another embodiment, the wells of the multiwell plates are coated with a mixture of two or more of the peptides.

In still another aspect of the invention, there is provided a method for forming an antibody enriched sera for use in treatment or prophylaxis of AIDS. According to this method a sample of body fluid, e.g. blood, from an individual testing positive for anti-p17 antibodies to HIV but which individual does not exhibit any symptoms of AIDS or ARC, is withdrawn from the individual and the presence of antibodies to the immunodominant epitopes of one or more of the peptides of formula (I), (II), (III), (IV) or (V) is determined. Then, one or more samples of the body fluid is subjected to affinity chromatography to remove any antibodies which do not confer protection against development of AIDS and/or to enrich the sera for any of such antibodies which do confer protection against development of AIDS.

## DETAILED DESCRIPTION OF THE INVENTION AND PREFERRED EMBODIMENTS

In addition to recent discoveries leading to the conclusion that HIV p17 is located near the surface of the HIV virion rather than in the internal core as in the case of the p24 gag protein, it has now been discovered that the p17 gag protein is myristylated at its N-terminal. Furthermore, analysis of the Peptide D (designated HGP-30), which is located nearer to the C-terminal of p17, suggests that this peptide contains an immunodominant B-cell epitope which could induce neutralizing antibodies and mediate cytotoxicity through the ADCC type mechanisms. On the other hand, the Peptide C, (also designated HGP-34) at amino acid positions 51-84, has an amino acid sequence consistent with a T-cell epitope, therefore capable of eliciting a T-cell immunological response. Analysis of the intermediate region Peptide B of p17 at positions 33-50 (also designated HGP-18), near the N-terminal suggests that this peptide has the appropriate balance of hydrophilicity and hydrophobicity to constitute a transmembrane region peptide if the appropriate charge neutralizing membrane proteins are present.

Based on the above observations, the present inventors have hypothesized that the HIV p17 protein extends across the lipid bilayer membrane of the entire HIV virion, or particle, and that the myristylated N-terminal region Peptide A (HGP-32) is anchored onto the membrane on its inner surface and extends below the inner surface, curving back to the membrane. The adjacent region Peptide B (HGP-18) extends across and through the membrane, i.e. is a transmembrane region peptide, and on the outer surface of the membrane, the regions Peptide C (HGP-34), Peptide D (HGP-30) and Peptide E (also designated Ala-HGP-17) (at the C-terminal) form a loop. The proposed orientation of p17 is schematically illustrated in Figure 1.

It should be understood, however, that applicants do not intend to be bound by this proposed orientation and other orientations may also be consistent with the observed data and amino acid sequences. For example, the p17 molecule may be located entirely inside the cell membrane, or entirely outside the cell membrane, or some proportion of all of the p17 molecules may be internal and the remaining p17 molecules external.

The amino acid sequence of p17 (strain HIVBH10) and the corresponding structures of the p17 protein of HIV-2 and simian immunodeficiency virus (SIV) are shown in Figure 2. In Figure 2, the "+" symbol indicates that the same amino acid is present at the relevant position in the respective proteins, the locations of the various peptide segments of the p17 HIV amino acid sequences being generally indicated in the margin. Also, for convenience, the amino acids of Peptide A (HGP-32) are underscored with a solid single line; the amino acids of Peptide B (HGP-18) are not underscored; the amino acids of Peptide C (HGP-34) are underscored with a single broken line; the amino acids of Peptide D (HGP-30) are underscored with a solid double line; and the amino acids of Peptide E (Ala-HGP-17) are underscored with a double broken line. From Figure 2, it is recognized that in each of HIV-1, HIV-2 and SIV the most conserved regions are towards the myristylated N-terminal, with increasingly lesser homology towards the C-terminal. However, over the region in Peptide D (HGP-30) of amino acids at positions 91-100 of p17 there is a highly conserved region with respect to both HIV-2 and SIV (about 40% homology) and, importantly, it is in this region that the immuno-dominant epitope and the major site towards which the neutralizing antibodies in heterologous antisera to HGP-30 are directed, as determined by studies carried out in rabbits.

Figure 3 illustrates the primary acid sequence of p17 of HIV-1, strain HB10, including the Peptides A, B, C, D and E designated as HGP-32, HGP-18, HGP-34, HGP-30 and Ala-HGP-17, respectively, wherein the numerical designations indicate the length of the specific p17 fragment. For convenience, in the peptide synthesis by solid phase peptide synthesis, as described below, variations in the naturally occurring amino acids of p17 have been made in some fragments for convenience in synthesis, but these analogs of the native p17 fragments are also within the scope of the invention. It will, of course, be appreciated that the designations of the five peptide fragments over the entirety of p17 is somewhat arbitrary, but consistent with the proposed orientation as shown in Figure 1. Often, the selection of N-terminal and C-terminal amino acids for each peptide was made for convenience of synthesis considerations, rather than based on biological activity, so long as the immunodominant epitope is retained. Variations in length of the amino acid sequences and substitution or addition or deletion of amino acids are permitted and within the scope of the invention.

In order to demonstrate the clinical importance of the five-contiguous regions making up the entirety of HIV p17 based on the above discussed model as illustrated in Figures 1, 2 and 3, small samples of analogs of the Peptides A, B, C and E and Peptide D' (HGP-18(2) - positions 91-108 of HIV-1 strain SF-2: Ile-Asp-Val-Lys-Asp-Thr-Lys-Glu-Ala-Leu-Glu-Lys-Ile-Glu-Glu-Glu-Gln-Asn) were prepared by solid phase peptide synthesis as described in the following examples. Using these samples, the presence of antibodies recognizing these synthetic peptides was first determined by exposing a rabbit immunized with native (intact) HIV p17 which had been purified by high pressure liquid chromatography (HPLC). The results are shown in Figure 4. For this rabbit the highest titers were for antibodies to Peptide C (HGP-34). Substantially no antibodies to Peptide B (51-Tyr) (HGP-18) were present and only weak titers for Peptide A (33-Lys) (HGP-32).

In another test, sera from four Rhesus Monkeys given live HIV-1 were also evaluated for antibodies to Peptides A, B, C, D and E and the results are shown in Figure 5. Each peptide had a strong antibody response in at least one of the challenged monkeys. Sera from individuals who had been naturally exposed to the HIV virus and were seropositive for p17 were also evaluated. The results are shown in the following Table II.

Again, as was the case in the treated monkeys, in each case, antibodies immunoreactive with at least one of the synthetic p17 peptide analogues, as measured by ELISA analysis, were present. Table II shows the results of the ELISA analyses for the HIV p17 seropositive individuals.

Table II

Presence of Antibodies to intact, natural p17 and p17 peptide analogs in HIV p17 seropositive individuals

| | | Peptide | | | | |
|---|---|---|---|---|---|---|
| | | E | D(86-Ser) | C(85-Lys) | B(51-Tyr) | A(33-Lys) |
| Patient No. | HIV p17 (1-131)* | HGP-17 (131-114) | HGP-30 (114-85) | HGP-34 (84-51) | HGP-18 (50-33) | HGP-32 (32-1) |
| 1 | + | ++ | ++ | - | + | - |
| 2 | ++ | ++ | ++ | + | + | - |
| 3 | + | + | ++ | + | + | ++ |
| 4 | ++ | ++ | ++ | + | - | + |
| 5 | ++ | + | + | + | - | - |
| 6 | ++ | ++ | - | - | + | ++ |
| 7 | ++ | + | - | - | - | - |
| 8 | ++ | + | - | - | - | - |
| 9 | ++ | + | - | - | - | - |
| 10 | ++ | ++ | - | - | ++ | - |
| 11 | ++ | + | - | - | + | ++ |
| 12 | ++ | + | - | - | + | - |
| 13 | ++ | - | ++ | + | - | - |

Notes: * numbers in parenthesis correspond to amino acid positions represented by the respective peptides. HIV p17 was measured by ELISA and Western Blot techniques
++ = greater than 3SD from mean
+ = greater than 2SD from mean

Since antibodies to all five peptides were present in at least 3 seropositive patients, each of these peptides, in addition to their utility as diagnostic reagents for determining exposure to HIV, also have potential benefits as sub-unit vaccines.

The peptides of the present invention are represented by the general formula (II)-(VI):

Positions 1-32

Gly-Ala-Arg-Ala-Ser-Val-Leu-Ser-Gly-Gly-
Glu-Leu-Asp-Arg-Trp-Glu-Lys-Ile-Arg-Leu-
Arg-Pro-Gly-Gly-Lys-Lys-Lys-Tyr-Lys-Leu-
Lys-His (II)

Positions 33-50

Ile-Val-Trp-Ala-Ser-Arg-Glu-Leu-Glu-Arg-
Phe-Ala-Val-Asn-Pro-Gly-Leu-Leu (III)

Positions 51-84

Glu-Thr-Ser-Glu-Gly-Cys-Arg-Gln-Ile-Leu-
Gly-Gln-Leu-Gln-Pro-Ser-Leu-Gln-Thr-Gly-
Ser-Glu-Glu-Leu-Arg-Ser-Leu-Tyr-Asn-Thr-
Val-Ala-Thr-Leu (IV)

Positions 114-131

Ala-Gln-Gln-Ala-Ala-Ala-Asp-Thr-Gly-His-
Ser-Ser-Gln-Val-Ser-Gln-Asn-Tyr (V)

including the analogues thereof wherein one or more of the amino acids in the sequence may be substituted, deleted or added, so long as the immunogenic reactivity to antibodies to HIV p17 derived from the 3-dimensional conformation of the sequence is preserved. Furthermore, it is understood, by convention, that the peptide formulas are given starting at the N-terminal on the left and ending with the C-terminal on the right.

Although not intending to be limited thereby, the types of "preserving" substitutions which would be expected to be interchangeable with each other while maintaining the immunogenic reactivity, include, for example, substitutions among the non-polar aliphatic neutral amino acids, including glycine (Gly, G), alanine (Ala, A), proline (Pro, P), valine (Val, V), isoleucine (Ile, I) and leucine (Leu, L); substitutions among the polar aliphatic neutral amino acids, including serine (Ser, S), threonine (Thr, T), methionine (Met, M), cysteine (Cys, C), asparagine (Asn, N) and glutamine (Gln, G); substitutions among the charged acidic amino acids, including aspartic acid (Asp, D) and glutamic acid (Glu, E); substitutions among the charged basic amino acids, including lysine (Lys, K) and arginine (Arg, R); and substitutions among the aromatic amino acids, including phenylalanine (Phe, F), histidine (His, H), tryptophan (Trp, W) and tyrosine (Tyr, Y), wherein the three letter groups and single letters in the parenthesis represent the conventional 3-letter and single letter codes for the respective amino acids.

More broadly, substitutable or interchangeable amino acids will generally often be found from among the foregoing groups as well, especially, for example, from among the following groups: Asp, Glu and His; Asn, Gln, Lys, and Arg; Asp, Glu, Lys, and Arg; Phe, His, Tyr, Trp, Asp, Glu, Ser, and Thr; Lys, Ile, Val, Leu, Pro, and Arg; Ser, Thr, Ala, Pro, and Val; and Gly, Ala, Pro, Asp, and Glu. Furthermore, among the aliphatic neutral non-polar amino acids preferred substitutions include Glu and Ala, and Val, Ile and Leu. Ser and Thr is also a preferred group of interchangeable amino acids.

When there are amino acids deleted from the peptides of formulas (II)-(V) or Peptide D (formula I) it is preferred that the deletions be taken from consecutive amino acids at the N- and/or C-terminals, preferably, symmetrically or nearly symmetrically from each of the N-terminal and C-terminal. Where there are internal deletions these should be preferably limited to 5, especially less than 3, such as 1 or 2, and when there are multiple internal deletions they may be from adjacent amino acids or from non-adjacent amino acids.

Similarly, when there are amino acid additions to the above peptides, it is preferred that the additions be made at the N- and/or C-terminals, preferably symmetrically or nearly symmetrically at each of the N-terminal and C-terminal. These additions may be the same as or different than the amino acids from the adjacent peptide fragment of the entire p17. However, internal additions may also often be permissible, but

when made, will preferably be limited to 5, especially to 3 or less, such as 1 or 2, and when more than one internal amino acid is added, they may be added consecutively (i.e. between adjacent amino acids) or non-consecutively (i.e. between non-adjacent amino acids).

Amino acid additions or substitutions or deletions at the N- and/or C-terminals are often especially useful since they may serve a variety of functions, such as a linking group, a modifying group, an identifying group (e.g. for radioimmunoassay, etc.) or the like. Often such substitutions, additions or deletions can simplify the synthesis of the particular peptide, such as by liquid or solid phase peptide synthesis as described below.

As specific examples of substitutions and additions the following modifications were implemented in Peptide A (HGP-32) of formula (II), Peptide B (HGP-18) of formula (III), and Peptide C (HGP-34) of formula (IV) to form the analogs used in the above-described test procedures:

Peptide A: C-terminal Lys addition
Peptide B: C-terminal Tyr addition
Peptide C: 56-Cys substitution by Ser and C-terminal Lys addition

In Peptide D (HGP-30) Cys-86 is replaced by Ser, i.e. formula (I).

The peptides of this invention can be prepared by conventional processes for synthesizing peptides; more specifically, using processes as described in Schroder and Lubke, The Peptides, Vol. 1 (1986), published by Academic Press, New York, U.S.A., or Izumiya et al., Synthesis of Peptides, (1975), published by Maruzen Publishing Co., Ltd., for example, an azide process, a chloride process, an acid anhydride process, a mixed anhydride process, a DCC process, an active ester process (a p-nitrophenyl ester process, an N-hydroxysuccinimide ester process, a cynaomethyl ester process, etc.), a process using a Woodward reagent K, a carbodiimidazole process, an oxidation reduction process, a DCC/additive (HONB, HOBt, HOSu) process, etc. Solid phase and liquid phase synthesis are both applicable to the foregoing processes.

The peptides of the present invention are prepared in accordance with the aforesaid processes for synthesizing ordinary polypeptides, generally either by a so-called stepwise process which comprises condensing an amino acid to the terminal amino acid one by one in sequence, or by coupling fragments divided into several groups to the terminal amino acid. In more detail, for example, in case the preferred solid phase synthesis is adopted, the C-terminal amino acid is bound to an insoluble carrier through its carboxyl group. The insoluble carrier is not particularly limited as long as it has a binding capability to a reactive carboxyl group and is inert to the reagents and reaction conditions of the stepwise condensation-deprotection reactions. Examples of such insoluble carriers include halogenomethyl resins such as chloromethyl resin, particularly chloromethylpolystyrene-divinylbenzene polymer, bromomethyl resin, etc., hydroxymethyl resins, phenol resins, tert-alkyloxycarbonylhydrazidated resins, crosslinked poly-N-acrylylpyrrolidine resins, etc. In case of, for example, the polypeptides wherein the C-terminal amino acid has an amide functional group, such as an asparagine (Asn),

$$NH_2 \cdot CH \cdot COOH,$$
$$CH_2 \cdot CONH_2$$

it is convenient to use a benzhydrylamine resin support, whereby the amide functional group can be directly formed on the resin support. Furthermore, according to a particularly preferred embodiment, the solid phase synthesis process for producing the peptides of formulas (I) and any other peptides in which the C-terminal amino acid includes an amide functional group, is carried out using methylbenzhydrylamine resin support as described in greater detail in the commonly assigned copending application of Su-Sun Wang, Serial No. 849,835, filed April 8, 1986, the disclosure of which is incorporated herein in its entirety by reference thereto.

After the amino protective group is removed, an amino group-protected amino acid is bound in sequence in accordance with the desired amino acid sequence as shown, for example, by general formula (I)-(V), through condensation of its reactive amino group and the reactive carboxyl group, in sequence, to synthesize step-by-step. After synthesizing the complete sequence, the peptide is split off from the insoluble carrier to produce the protein, using, for example, HF, HBr, or other cleavage agent.

In the description above and following, the abbreviations have the following meaning according to standard customary nomenclature:

Boc, t-butyloxycarbonyl, Bzl, benzyl; DCC, dicyclohexylcarbodiimide; Z, benzyloxycarbonyl; TFA, trifluoroacetic acid, and ClZ, 2-chlorobenzyloxycarbonyl.

While specific protecting groups are described below, it is within the skill of the art to utilize equivalent conventional protecting groups. The practitioner will readily recognize that certain general conditions will preferably be selected regardless of the particular peptide being synthesized. For example, it is well recognized that the α-amino group of each amino acid employed in the peptide synthesis must be protected during the coupling reaction to prevent side reactions involving the reactive α-amino function. Similarly, for those amino acids containing reactive side-chain functional groups (e.g. sulfhydryl, ϵ-amino, hydroxyl, carboxyl), such functional groups need also be protected both during the initial coupling of the amino acid containing the side-chain group and during the coupling of subsequent amino acids. Suitable protecting groups are known in the art. [See, for example, Protective Groups in Organic Chemistry, M. McOmie, Editor, Plenum Press, N.Y., 1973.]

In selecting a particular protecting group, the following conditions must be observed: and α-amino protecting group must: (1) be stable and render the α-amino function inert under the conditions employed in the coupling reaction, and (2) must be readily removable after the coupling reaction under conditions that will not remove the side chain protecting groups or alter the structure of the peptide fragment. A side-chain protecting group must: (1) be stable and render the side-chain functional group inert under the conditions employed by the coupling reaction, (2) be stable under the conditions employed in removing the α-amino protecting group and (3) be readily removable upon completion of the desired amino acid sequence under reaction conditions that will not alter the structure of the peptide chain, or racemization of any of the chiral centers contained therein. Suitable protecting groups for the α-amino function are t-butyloxycarbonyl (Boc), benzyloxycarbonyl (Z), biphenylisopropyloxycarbonyl, t-amyloxycarbonyl, isobornyloxycarbonyl, 1,1-dimethyl-2,5-dimethoxybenzyloxycarbonyl, o-nitrophenylsulfenyl, 2-cyano-t-butyloxycarbonyl, 9-fluorenylmethyloxycarbonyl and the like, especially t-butyloxycarbonyl (Boc).

As examples of carboxyl-protecting groups, there may be mentioned such ester-forming groups as those capable of giving alkyl ester (e.g. methyl, ethyl, propyl, butyl, t-butyl, etc., esters), benzyl ester, p-nitrobenzyl ester, p-methoxybenzyl ester, p-chlorobenzyl ester, benzhydryl ester, etc. and hydrazide-forming groups such as those capable of giving carbobenzoxy hydrazide, t-butyloxycarbonyl hydrazide, trityl hydrazide, etc.

As groups for protecting the guanidino group of arginine, there may be mentioned nitro, tosyl (Tos), p-methoxybenzenesulfonyl, carbobenzoxy, isbornyloxycarbonyl, admantyloxycarbonyl, etc. The guanidino group may also be protected in the form of a salt with an acid (e.g. benzenesulfonic acid, toluenesulfonic acid, hydrochloric acid, sulfuric acid, etc.).

The hydroxyl group of threonine may be protected, for example, by way of known esterification or etherification. As examples of groups suitable for said esterification, there may be mentioned lower alkanoyl groups (e.g. acetyl), aroyl groups (e.g. benzoyl), and groups derived from carbonic acid, such as benzyloxycarbonyl, ethyloxycarbonyl, etc. As groups suitable for said etherification, there may be mentioned benzyl, tetrahydropyranyl, t-butyl, etc. The hydroxyl group of threonine, however, need not necessarily be protected. Methionine may be protected in the form of a sulfoxide. Other preferred side-chain protective groups for particular amino acids include; for tyrosine: benzyl, o-bromobenzyloxycarbonyl (BrZ), 2,6-dichlorobenzyl, isopropyl, cyclohexyl, cyclopentyl and acetyl; for histidine: benzyl, benzyloxycarbonyl, p-toluenesulfonyl, trityl and 2,4-dinitrophenyl (DNP); for tryptophan: formyl.

As typical procedures for the initial coupling of the C-terminal amino acid to the solid resin support the following guidelines are mentioned. For benzhydrylamine or methylbenzhydrylamine resin, the $N^{\alpha}$-Boc-amino acid or similarly protected C-terminal amino acid is attached to the (methyl)benzhydrylamine resin by means of an N,N'-dicyclohexylcarbodiimide (DCC)/1-hydroxybenzotriazole(HBT) mediated coupling for from about 2 to 24 hours, preferably about 12 hours at a temperature of between about 10° and 50°C., preferably 25°C., in a solvent such as dichloromethane or DMF, preferably dichloromethane. For chloromethyl polystyrenedivinylbenzene type of resin: The attachment to the resin is made by means of the reaction of the $N^{\alpha}$-protected amino acid, especially the Boc-amino acid, as its cesium, tetramethylammonium, triethylammonium, 4,5-diazabicyclo[5.4.0]-undec-5-ene, or similar salt in ethanol, acetonitrile, N,N-dimethylformamide (DMF), and the like, especially the cesium salt in DMF, with the chloromethyl resin at an elevated temperature, for example, between about 40° to 60°C., preferably about 50°C., for from about 12 to 48 hours, preferably about 24 hours.

The removal of the $N^{\alpha}$-protecting groups may be performed in the presence of, for example, a solution of trifluoroacetic acid in methylene chloride, hydrogen chloride in dioxane, hydrogen chloride in acetic acid, or other strong acid solution, preferably 50% trifluoroacetic acid in dichloromethane at about ambient temperature. The coupling of successive protected amino acids can be carried out in an automatic polypeptide synthesizer, as well known in the art. Each protected amino acid is preferably introduced in approximately 2.5 or more molar excess and the coupling may be carried out in dichloromethane,

dichloromethane/DMF mixtures, DMF and the like, especially in methylene chloride at about ambient temperature. Other solvents which are known to be useful for the purpose of peptide-forming condensation reaction, for example, dimethylsulfoxide, pyridine, chloroform, dioxane, tetrahydrofuran, ethyl acetate, N-methylpyrrolidone, etc., as well as suitable mixtures thereof, may also be used.

The reaction temperature for the condensation/coupling reaction may be selected from the range known to be useful for the purpose of peptide-forming condensation reactions. Thus, it may normally be within the range of about -40°C. to about 60°C., and preferably, about -20°C. to about 0°C. The coupling agent is normally DCC in dichloromethane but may be N,N'-diisopropylcarbodiimide or other carbodiimide either alone or in the presence of HBT, N-hydroxysuccinimide, ethyl 2-hydroxyimino-2-cyanoacetate, other N-hydroxyimides or oximes. Alternatively, protected amino acid active esters (e.g. p-nitrophenyl, pentafluorophenyl and the like) or symmetrical anhydrides may be used.

The coupling, deprotection/cleavage reactions and preparation of derivatives of the polypeptides are suitably carried out at temperatures between about -10° and +50°C., most preferably about 20°-25°C. The exact temperature for any particular reaction will, of course, be dependent upon the substrates, reagents, solvents and so forth, all being well within the skill of the practitioner. The fully deprotected polypeptide may then be purified by a sequence of chromatographic steps employing any or all of the following types: ion exchange on a weakly basic resin in the acetate form; gel permeation chromatography, e.g. on Sephadex G-25; hydrophobic adsorption chromatography on underivatized polystyrene-divinylbenzene (for example, Amberlite XAD); silica gel adsorption chromatography; ion exchange chromatography, e.g. on Sephadex G-25, or counter-current distribution; high performance liquid chromatography (HPLC), especially reverse phase HPLC on octyl-or octadecylsilylsilica bonded phase column packing.

Although the synthetic peptides according to this invention can be prepared by the above-described chemical preparations, especially the solid phase peptide synthesis, described above, with high efficiency, it is also within the scope of the invention to produce the novel HIV p17 peptide fragments or analogs thereof, by genetic engineering technology, as is now well known in the art. Thus, using appropriate enzymes and microorganisms (e.g. bacteria, such as E. coli) the DNA sequence encoding for the desired polypeptide can be incorporated into the genome of the microorganism, thereby causing the microorganism to express the particular peptide of interest.

Once the particular peptide is obtained and purified, it may be used as a hapten to prepare an antigenic immunogen and from the antigen, antibodies having specific cross-reactivity with the p17 gag protein of HIV and similar viruses, and having neutralizing capability for the AIDS virus can readily be obtained in large amounts and stably. The specific antibodies can, therefore, be used as antisera for inhibiting replication of the AIDS virus. The antibodies can be used for purification of immunoreactive proteins in HIV by binding these antibodies to carriers for use in, e.g. affinity chromatography. The specific antibodies can also be used as specific antibodies in various immunological measurements of the different strains of HIV retroviruses. Therefore, the antibodies according to this invention are valuable for directly diagnosing, with high specificity, the presence in blood sera of the AIDS virus.

As stated above, antibodies having neutralizing capacity for HIV can be obtained by immunizing a healthy normal patient or animal with the p17 peptides of this invention. However, it is sometimes the case that antibodies to particular viral fragments may actually be harmful, for example, by facilitating entry of the antibody-bound virus to penetrate into the target cells. Such harmful antibodies have, in fact, been suggested in the literature for the AIDS virus, particularly antibodies to certain envelope proteins.

Accordingly, it is an important feature of this invention to screen out any harmful or potentially harmful antibodies to the invention peptides, as well as enriching for desired antibodies. The removal of undesired peptides and/or purification or enrichment of desirable peptides can be effected using the technique of affinity chromatography. As used in this context affinity chromatography consists of binding an antigen (e.g. peptides or analogs of this invention) to an insoluble matrix and then passing the sera of the immunized subject through a column filled with the bound antigen. Thereafter, by appropriate elution unwanted antibodies can be removed and desirable antibodies can be enriched.

The techniques for coupling peptides to insoluble matrix and subsequent elution used in affinity chromatography are well known in the art and any of these available methods applicable to peptides and proteins can be used. For example, according to one preferred procedure for coupling the peptide to an insoluble matrix (cyanogen bromide-activated Sepharose 4B -- from Pharmacia, N.J.) the insoluble matrix is activated with $10^{-3}$M HCl (1 gram/200 ml). The activated matrix is washed twice with a coupling buffer (e.g. 0.1 m $NaHCO_3$, 0.5 M NaCl, pH = 8.0) and an amount of the matrix, in the form of a gel, sufficient to provide 1 gram gel for each 5 mg peptide is aliquoted and pelleted in a centrifuge tube (e.g. at 1200 r.p.m.). The peptide to be coupled is solubilized in coupling buffer, added to the centrifuge tube, and gently rocked for about 8 to 20 hours at 40°C. The peptide coupled matrix (Sepharose) is washed 3 times each with acidic

(e.g. 0.1 M acetate, 1 M NaCl, pH = 8.0) and basic (e.g. 0.1 M bicarbonate, 1 M NaCl, pH = 8.0) buffers.

Removal of undesired antibodies (to the bound peptide) from the subject antisera can be accomplished using the Sepharose bound peptide as follows: The Sepharose peptide complex is placed in a glass column and 10 ml of antisera in buffer (e.g. phosphate buffered saline - PBS, pH = 7.4) is added to the column. The column is then washed with 20 ml of PBS buffer and eluent collected in 1 ml aliquots. The eluent will contain antibodies which did not bind to the peptide.

Conversely, if the bound peptide is one which elicits desired antibodies, such that enrichment of the bound antibodies is the goal, then removal of the bound antibodies may be accomplished, for example, by filling the column with 0.1 M glycine, pH = 2 as buffer. The column is then washed with 20 ml of PBS buffer and contain the desired antibodies which did not bind to the peptide. Neutralization of the eluent can be effected by adding to each tube 50 l of 1M Tris = HCl, pH 9.5.

Polypeptides displaying the antigenicity of the p17 gag protein and antibodies raised with them can be employed in these diagnostic methods to detect the presence of, respectively, antibodies to the AIDS virus or the AIDS virus, per se. These polypeptides and their antibodies may be packaged in diagnostic kits either alone or in combination with any or all of the other HIV antigens and antibodies. These methods and kits, using polypeptides or antibodies of this invention either alone or in combination with other antigens or antibodies, will allow the rapid and convenient identification of AIDS infective carriers and prediction of the course of ARC and AIDS disease. Brief descriptions of typical radioimmunoassay and ELISA for detecting AIDS virus are given below.

Radioimmunoassay to detect AIDS virus: Antibodies to the antigenic determinant of the amino acid sequence of formula (I)-(V) of the p17 gag protein of the AIDS virus produced as above are attached to a solid phase, for example, the inside of a test tube. A sample of the patient's serum is added to a tube, together with a known amount of polypeptides displaying the antigenicity of p17 gag protein of the AIDS virus produced as above and labelled with a radioactive isotope such as radioactive iodine. Any AIDS virus (with associated gag proteins) in the patient's serum will compete with the labelled polypeptides displaying the antigenicity of the p17 gag protein of the AIDS virus for binding with the bound antibodies. The excess liquid is removed, the test tube washed, and the amount of radioactivity measured. A positive result, i.e. that the patient's serum contains AIDS virus, is indicated by a low radioactive count left in the tube. Instead of the solid phase RIA as described above it may be more convenient in some cases to perform liquid phase RIA. In the case of liquid phase RIA the antibodies recognizing the AIDS virus, e.g. anti-peptide (I), anti-peptide (II), anti-peptide (III), etc., are not attached to a solid phase, but are allowed to react in the liquid phase with the patient's serum in competition with the labelled antigenic material. The antibody-antigen interaction complex can then be detected by any suitable means. For example, a second antibody reactive to the first antibody (such as an anti-antibody raised to the first antibody but in a different animal) bound to an insoluble bead can be used to withdraw the complex from the liquid phase.

ELISA to detect antibodies to the AIDS virus: a microtiter plate is coated with a peptide prepared in accordance with this invention displaying the antigenicity of the p17 gag protein of the AIDS virus, and to this is added a sample of patient's serum. After a period of incubation permitting interaction between the bound peptide and any p17 gag antibody to the AIDS virus present in the serum, the plate is washed. A preparation of p17 peptide, which can be the whole peptide or the same peptide fragment as on the plate (e.g. a so-called "sandwich"), linked to an enzyme, is added. Incubation allows an antibody-antigen reaction to take place, and the plate is then rewashed. Thereafter, enzyme substrate is added to the microtiter plate and incubated for a period of time to allow the enzyme to work on the substrate, and the absorbance of the final preparation is measured. A large change in absorbance indicates a positive result.

The details of the radioimmunoassays and ELISA as generally outlined above for measuring antigens are well known in the art.

The peptides of the present invention are utilizable as labelled antigens employed in radioimmunoassay (RIA) or enzyme immunoassay (EIA), particularly enzyme linked immunosorbent assay (ELISA), by introducing thereto radioactive substances such as $^{125}I$, $^{131}I$, $^{3}H$ (tritium), $^{14}C$, etc.; various enzyme reagents such as peroxidase (POX), chymotrypsinogen, procarboxypeptidase, glyceraldehyde-3-phosphate dehydrogenase, amylase, phosphorylase, D-Nase, P-Nase, $\beta$-galactosidase, glucose-6-phosphate dehydrogenase, ornithine decarboxylase, etc. The introduction of the above radioactive substance can be effected in a conventional manner. For example, the introduction of radioactive iodine, $^{125}I$, can be carried out by the oxidative iodination method using chloramine T (W. M. Hunter and F. C. Greenwood, Nature, 194, 495 (1962), Biochem. J., 89, 144 (1963)), or by using the Bolten-Hunter reagent ($^{125}I$-iodinated p-hydroxyphenyl propionic acid N-hydroxy-succinimide ester) as described in Biochem. J., 133, 529 (1973). See Example 2 in U.S. Patent 4,264,571, etc.

More particularly, this method can be carried out in an appropriate solvent, e.g. 0.2M phosphate buffer solution (pH 7.4) in the presence of chloramine T at about room temperature for about 10 to 30 seconds. The peptide, radioactive iodine 125 and chloramine T are used in ratios of about 1mCi of the radioactive iodine per nmol of tyrosine contained in the peptide and 10 to 100 mnmol of chloramine T per nmol of tyrosine contained in the peptide when introducing one atom of the radioactive iodine per molecule of tyrosine, and about 2mCi of the radioactive iodine per nmol of tyrosine contained in the peptide and 10 to 100 nmol of chloramine T per nmol of tyrosine contained in the peptide when introducing two atoms of the radioactive iodine per molecule of tyrosine. The thus prepared radioactive iodine labelled peptides can be isolated and purified from the reaction mixture by a conventional separation techniques e.g. extraction, distribution, column chromatography, dialysis, etc. The thus obtained peptides can be stored after freeze drying, if desired.

For the peptides of formula (II) or (V) wherein Tyr is included in the amino acid sequence or for the peptide analogues of formula (III) or (IV) in which a Tyr is added or substituted $^{125}I$ can be directly incorporated into the Tyr, such as by, for example, the procedure described in Example 3b of U.S. Patent 4,339,427.

The introduction of enzyme reagents can be conducted by known method such as conventional coupling reactions, e.g. the B. F. Erlanger, et al. method (Acta Endocrinol. Suppl., 168, 206 (1972)), the M. H. Karol, et al. method (Proc. Natl. Acd. Sci. U.S.A., 57 713 (1967)), etc. That is, a peptide and an enzyme are reacted in a buffer solution having a pH of 4 to 6, e.g. 1mM acetate buffer (pH 4.4) in the presence of an oxidizing agent such as $NaIO_4$ at about room temperature for 2 to 5 hours, followed by reduction with $NaBH_4$ or the like. The enzyme can be used in an amount of 1 to 3 mols per moles of the peptide. It is preferred that the oxidizing agent be used in an amount of about 100 to about 300 mols per mol of the peptide and the reducing agent be used in an amount of about 1 to about 2 mols per mol of the oxidizing agent. The thus obtained enzyme-labelled peptide can be isolated, purified and stored in a manner similar to the processes used in the case of the radioactive iodine-labelled peptide.

It is recognized in the art that polypeptides, i.e. peptides containing less than about 50 amino acids, will not generally directly elicit production of a corresponding antibody. For this reason, it is the usual practice to couple or conjugate the polypeptide antigen, as a hapten, to a hapten-carrier, hereinafter referred to as "immunogenic carrier material" to thereby elicit the production of specific antibodies in sufficiently high titers to be easily recoverable. In the present invention, while it is generally preferred to use the HIV p17 gag peptides (including analogs) as haptens, coupled to an immunogenic carrier material, it is also within the scope of the invention, particularly for the peptides with at least about 26 amino acids, to directly use the peptides as vaccines or as immunogenic antigens in the production of specific antibodies, as well as, of course, in the diagnostic aspects of the invention. Still further, it is within the scope of the invention to utilize any of the novel peptides in the form of its "polymer", such as dimer, trimer or oligomer, that is as repeating sequences containing the amino acids of the synthetic peptide. Thus, the synthetic peptides can be polymerized to build up a chain of two or more repeating units so that repeating sequences serve both as "carrier" and immunologically active site.

Hereafter, processes for production of immunogenic antigens using the peptides of the present invention as haptens will be described in detail.

The aforementioned antigens are prepared by using the peptides of the present invention as haptens and reacting the peptides with a suitable immunogenic carrier material in the presence of a hapten-carrier binding agent. In this case, natural and synthetic proteins having a high molecular weight which are conventionally employed in the preparation of antigens can be widely employed as carriers to be bound to haptens. In addition, smaller peptides or molecules, such as tufsin (a tetrapeptide), analogs of tufsin, and muramyl dipeptides, and their analogs can act equally well as "immunogenic carrier materials."

As used herein the term "immunogenic carrier material" is meant to include those materials which have the property of independently eliciting an immunogenic response in a host animal and which can be covalently coupled to polypeptide either directly via a formation of peptide or ester bonds between free carboxyl, amino or hydroxyl groups in the polypeptide and corresponding groups on the immunogenic carrier material or alternatively by bonding through a conventional bifunctional linking group. Examples of such carriers include albumins of animal sera such as horse serum albumin, bovine serum albumin, rabbit serum albumin, human serum albumin, sheep serum albumin, etc.; globulins of animal sera such as horse serum globulin, bovine serum globulin, rabbit serum globulin, human serum globulin, sheep serum globulin, etc.; thyroglobulins of animals such as horse thyroglobulin, bovine thyroglobulin, rabbit thyroglobulin, human thyroglobulin, sheep thyroglobulin, etc.; hemoglobulins of animals such as horse hemoglobulin, bovine hemoglobulin, rabbit hemoglobulin, human hemoglobulin, sheep hemoglobulin, etc.; hemocyanins of animals such as Keyhole limpet hemocyanin (KLH), etc.; proteins extracted from ascaris (ascaris extracts, such

as those described in Japanese Patent Application (OPI) No. 16,414/81, J. Immun., 111 260-268 (1973), ibid., 122, 302-308 (1979), ibid., 98,893-900 (1967) and Am. J. Physiol., 199, 575-578 (1960), or purified products thereof); polylysine, polyglutamic acid, lysine-glutamic acid copolymers, copolymers containing lysine or ornithine, etc. Recently, vaccines have been produced using diphtheria toxoid or tetanus toxoid as immunogenic carrier materials (see Lepow. M.L., et al., J. of Infectious Diseases, Vol. 150, No. 3, pages 402-406 (1984); and Coen Beuvery, E., et al., Infection and Immunity 40, pages 39-45 (April 1983) and these toxoid materials can also be used herein. Other suitable carriers are disclosed in, for example, U.S. Patent 4,575,495, including viruses, e.g. vaccinia virus, organic polymers, etc.

As hapten-carrier binding agents, those conventionally employed in the preparation of antigens can be widely employed. Specific examples of these agents include diazonium compounds for crosslinking tyrosine, histidine, tryptophane, etc., e.g. bisdiazotized benzidine (BDB), etc.; aliphatic dialdehydes for crosslinking an amino group with an amino group, e.g. $C_2$-$C_7$ alyanals, such as glyoxal, malonedialdehyde, succinaldehyde, adipaldehyde, etc.; dimaleimide compounds for crosslinking a thiol group with a thiol group, e.g. N,N'-o-phenylenedimaleimide, N,N'-m-phenylenedimaleimide, etc.; maleimidocarboxyl-N-hydroxysuccinimide esters for crosslinking an amino group with a thiol group, e.g. metamaleimidobenzoyl-N-hydroxysuccinimide ester, 4-(maleimidomethyl)-cyclohexane-1-carboxyl-N'-hydroxysuccinimide ester, etc.; agents used in conventional peptide bond forming reactions in which amide bonds are formed from an amino group and a carboxyl group, e.g. dehydrating and condensing agents such as carbodiimides, e.g. N,N'-dicyclohexylcarbodiimide, N-ethyl-N'-dimethylamino-carbodiimide, 1-ethyl-3-diisopropylaminocarbodiimide, 1-cyclohexyl-3-(2-morpholinyl-4-ethyl)-carbodiimide, etc. As the foregoing hapten-carrier binding agent, it is also possible to use diazonium aryl carboxylic acids such as p-diazonium phenylacetic acid, etc., with conventional peptide bond forming agents such as the dehydrating and condensing agents described above in combination.

The covalent coupling of an acidic peptide to the immunogenic carrier material can be carried out in a manner well known in the art. Thus, for example, for direct covalent coupling it is possible to utilize a carbodiimide, most preferably dicyclohexylcarbodiimide or 1-ethyl-3-(3-dimethylaminopropyl)carbodiimide as coupling agent. In such direct coupling it is desirable to utilize a slightly acidic reaction medium for this step, e.g. a medium having a pH in the range of from about 3 to 6.5, most preferably in the range of from about 4 to about 6.5.

The coupling reaction for preparing antigens using as haptens peptides which are neutral or alkaline can be carried out in a similar manner but in an aqueous solution or conventional buffer solution having pH of 7 to 10, preferably in a buffer solution having pH of 8 to 9, at temperatures of about 0° to 40°C., preferably around room temperature. The reaction is generally completed within about 1 to about 24 hours, preferably 3 to 5 hours. Representative examples of buffer solutions which can be used in the above process include:

0.2M sodium hydroxide-0.2M boric acid-0.2M potassium chloride buffer solution;

0.2M sodium carbonate-0.2M boric acid-0.2M potassium chloride buffer solution;'

0.05M sodium tetraborate-0.2M boric acid-0.05M sodium chloride buffer solution;

0.1M dihydrogen potassium phosphate-0.05 sodium tetraborate buffer solution.

In the above, proportions of the hapten, hapten-carrier binding agent and carrier can be appropriately determined but it is preferred that the carrier be employed in an amount of about 1 to about 6 times, preferably about 1 to about 5 times the weight of the hapten and the hapten-carrier binding agent be employed in an amount of about 5 to about 10 times the mol of the hapten. By the above reaction, the carrier is bound to the hapten via the hapten-carrier binding agent to obtain a desired antigen composed of a peptide-carrier complex.

After completion of the reaction, the thus obtained antigen can easily be isolated and purified by means of a dialysis method, a gel filtration method, a fractionation precipitation method, etc.

The thus obtained antigen binds 5 to 60 mols, in average, of the peptide thereto per mole of a protein and enables one to subsequently prepare an antibody having a high specificity to the antigen. Particularly, antigens to which the peptide is bound in an amount of 5 to 20 mols, preferably 8 to 15 mols, in average, per mole of a protein are preferred since they have higher specificity and enable one to obtain antibodies having high activity and sensitivity.

The preparation of an antibody using the antigen is carried out by administering the aforesaid antigen, preferably using an adjuvant, to mammals to thereby produce a desired antibody in vivo and collecting the antibody. Improved titers can be obtained by repeated injections over a period of time.

As examples of adjuvants mention can be made, for example, to alum, Freund's adjuvant (complete or incomplete), defined and purified oils, such as Monomide A271 or squalene, bacterial extracts (e.g. RIBI), liposomes, ISCOMS, and the like.

While there is no particular limitation to mammals provided for the preparation of antibodies, it is generally preferred to use rabbits or guinea pigs, but horses, goats, pigs, rats, cows, sheep, etc., can also be used. In the production of antibodies, a definite amount of the antigen obtained as described above is diluted with a physiological saline solution to a suitable concentration and the resulting dilution is mixed with a complete Freund's adjuvant to prepare a suspension. The suspension is administered to mammals. For example, the aforesaid suspension is subcutaneously administered (0.1 to 5 mg/time as the amount of the antigen) to rabbit. Then the suspension is administered every two weeks over a period of 2 to 10 months, preferably 4 to 6 months, to effect immunization. The collection of the antibody is carried out by collecting blood from the immunized animal after the passage of 1 to 2 weeks subsequent to the final administration, centrifuging the blood and isolating serum from the blood. According to this procedure, an antibody which will selectively complex to the antigenic determinant of the AIDS virus, particularly the p17 gag protein, can be collected and used as an antisera for immunizing a patient at risk for AIDS (e.g. homosexuals, Haitians, intravenous drug users, and so on), exposed to the AIDS virus,, or suffering from the symptoms of AIDS or ARC. Alternatively, the antibodies can be used for assaying for virtually any of the known strains of HIV, especially HIV-1 and also HIV-2, capable of causing AIDS, utilizing RIA, ELISA, etc. For example, when used in a serological assay the antibodies of the present invention can be labelled with a radioactive isotope for use in an RIA or with enzymes or fluorescent substances conventionally used and can be used in ELISA, fluorescent immunoassay (FIA), etc. Further, the antibodies of the present invention can be insolubilized by reacting them with a conventional insolubilizing agent.

The present invention, therefore, provides antigenic p17 gag peptides and mixtures thereof, including the analogs thereof, which should provide powerful vaccines for neutralizing AIDS virus. Therefore, the vaccines of this invention can be used to immunize patients at risk for AIDS, or exposed to the AIDS virus or to treat patients with AIDS-Related Complex or with frank AIDS.

When used as a vaccine, the vaccine can be introduced into the host most conveniently by injection, intramuscularly, parenterally, orally or subcutaneously. Any of the common liquid or solid vehicles may be employed, which are acceptable to the host and which do not have any adverse side effects on the host nor any detrimental effects on the vaccine. Phosphate buffered saline (PBS), at a physiological pH, e.g. pH 6.8 to 7.2, preferably pH 7, may be used as a carrier, alone or with a suitable adjuvant. The concentration of immunogenic antigen may vary from about 50 to 200 $\mu$g, such as about 100 $\mu$g per injection, in a volume of solvent generally of from about 0.25 to 1, such as about 0.5 ml. Multiple injections may be required after the initial injections and may be given at intervals of from about 2 to 14 days, for example, about 7 days, when multiple injections are given.

As an alternative to the above described active immunization in which the administration to a host is of the immunogenic antigenic peptide, it is also possible to directly administer to a host the anti-AIDS virus antibodies of this invention, usually raised in a different species than the host, although antibodies raised in a different individual of the same species as the host may also be used. In the case of such passive immunizing antisera, generally the same modes of application and the same types of carriers and the same or higher dosage rates as described for the active immunization method can also be used. For example, as much as 1.5 grams of murine monoclonal antibody can be administered slowly (e.g. about 6 hours) by intravenous infusion.

Of particular importance in this regard is the ability to utilize the individual fragments of formulas (I)-(VI) or portions or analogs thereof retaining the immunodominant epitopes of HIV p17 to isolate and remove unwanted, potentially harmful antibodies, or to isolate and enrich for desired (e.g. neutralizing) antibodies to HIV. Thus, as described above, with the peptides of this invention it becomes possible to treat sera from HIV infected, healthy individuals (or normal, non-infected individuals previously vaccinated with the peptide fragments according to the invention and comprising the immunodominant epitopes of intact HIV p17) such that protective antibodies are present in the sera, using affinity chromatography, to remove any unwanted antibodies and/or enrich for desired helpful antibodies.

Still another alternative embodiment is a vaccine and immunization method based on an anti-idiotypic antibody raised against a monoclonal antibody to any one of the peptides of the invention. Anti-immunoglobin sera raised against a monoclonal antibody is defined as "anti-idiotype" serum and recognizes the variable region of the eliciting antibody and is directed against the combining site. This anti-idiotype antibody which is directed against the combining site can, in fact, substitute for the original antigen. Therefore, it is possible to use this mechanism to immunize and raise antibodies against the p17 gag peptide fragments and analogs as, or more, efficiently than via classic immunization protocols.

The synthesis of representative peptides according to this invention will now be shown by the following examples.

Example 1 - Preparation of Peptide B (51-Tyr)

Ile-Val-Trp-Ala-Ser-Arg-Glu-Leu-Glu-Arg-Phe-Ala-Val-Asn-Pro-Gly-Leu-Leu-Tyr

Boc-Tyr(BrZ)-$OCH_2$-$C_6H_4$-Resin (2.0 g; 0.9 mmol) was placed in a 100 ml peptide synthesis flask and solid phase synthesis commenced according to the following schedule (20 volumes of solvent or reagents used in each step) in each cycle:

1. three washings with $CH_2Cl_2$,
2. prewash with 50% TFA (in $CH_2Cl_2$),
3. stir 30 min in 50% TFA,
4. three washings with $CH_2Cl_2$,
5. prewash with 10% tributylamine ($Bu_3N$) (in $CH_2Cl_2$)
6. stir 3 min in 10% $Bu_3N$,
7. three washings with $CH_2Cl_2$,
8. stir 60 min with 2.7 mmol each of Boc-Leu (0.67 g) and DCC (0.556 g) in $CH_2Cl_2$,
9. wash once with $CH_2Cl_2$,
10. two washings with 50% isopropylalcohol (i-PrOH) in $CH_2Cl_2$,
11. three washings with $CH_2Cl_2$,
13. check ninhydrin reaction of the resin; if positive, repeat steps 8-13; if negative, go to next cycle.

The synthetic cycle was repeated using the following Boc-amino acids, sequentially, one at a time in step 8:

Boc-Leu, Boc-Gly, Boc-Pro, Boc-Asn, Boc-Val, Boc-Ala, Boc-Phe, Boc-Arg(Tos), Boc-Glu(OBzl), Boc-Leu, Boc-Glu(OBzl), Boc-Arg (Tos), Boc-Ser(Bzl), Boc-Ala, Boc-Trp, Boc-Val, and Boc-Ile.

The completed resin,

Boc-Ile-Val-Trp-Ala-Ser(Bzl)-Arg(Tos)-Glu(OBzl)-Leu-Glu(OBzl)-Arg(Tos)-Phe-Ala-Val-Asn-Pro-Gly-Leu-Leu-Tyr(BrZ)-$OCH_2$-$C_6H_4$-resin

was then treated with anhydrous HF (0°, 45 min) to deprotect and cleave the peptide from the resin. From 1.0 g of protected resin, 0.33 g of crude peptide was obtained. Part of the crude material (0.16 g) was purified on semi-preperative HPLC (Hamilton PRP-1 2.15 x 25 cm column; pH 4, potassium phosphate buffer, 23%/28% acetonitrile) to provide 18 mg of desired resin. It was shown to be homogeneous by analytical HPLC.

Amino Acid Anal. Asp, 1.10; Ser, 1.10; Glu, 2.04; Pro, 0.93; Gly, 1.08; Ala, 1.85; Val, 1.63; Ile, 0.86; Leu, 3.00; Tyr, 0.93; Phe, 0.94; Trp, 0.64 (acid hydrolysis); Arg, 1.94.

Example 2 - Peptide (I') at Positions 91-100

Ile-Glu-Ile-Lys-Asp-Thr-Lys-Glu-Ala-Leu

Boc-Leu-$OCH_2$-$C_6H_4$-Resin (1.0 g; 0.5 mmol) was placed in the peptide synthesis flask and the cycles of the solid phase peptide synthesis carried out according to the schedule described above in Example 1 with the following BOC-amino acids (1.5 mmol each) sequentially coupled to the peptide chain on the resin, one amino acid derivative per cycle:

BOC-Ala, Boc-Glu(OBzl), Boc-Lys(ClZ), Boc-Thr(Bzl), Boc-Asp(OBzl), Boc-Lys(ClZ), Boc-Ile, Boc-Glu-(OBzl) and Boc-Ile.

The completed resin,

Boc-Ile-Glu(OBzl)-Ile-Lys(ClZ)-

Asp(OBzl)-Thr(Bzl)-Lys(ClZ)-Glu(OBzl)-Ala-Leu-$OCH_2$-$C_6H_4$-Resin

thus obtained weighed 1.7 g. Part of this material (0.88 g) was then treated with anhydrous HF at 0° for 45 min and worked up to provide 0.27 g of crude peptide. Purification of 38 mg of this crude peptide of HPLC yielded 20 mg of desired peptide. It was shown to be homogeneous by analytical HPLC.

Amino Acid Anal. Asp, 1.00; Thr, 0.91; Glu, 2.08; Ala, 0.90; Ile, 1.92; Leu, 1.08; Lys, 2.00.

Example 3 - Preparation of Peptide E (Ala-HGP-17)

Ala-Gln-Gln-Ala-Ala-Ala-Asp-Thr-Gly-His-Ser-Ser-Gln-

Val-Ser-Gln-Asn-Tyr

Boc-Tyr(BrZ)-$OCH_2$-$C_6H_4$-Resin (1.0 g; 0.5 mmol) was placed in a peptide synthesis flask and the cycles of the solid phase peptide synthesis carried out according to the schedule described above in Example 1 with following Boc-amino acids (1.5 mmol each) sequentially coupled to the growing peptide chain on the resin, one amino acid derivative per cycle:

Boc-Asn, Boc-Gln, Boc-Ser(Bzl), Boc-Val, Boc-Gln, Boc-Ser(Bzl), Boc-Ser(Bzl), Boc-His(DNP), Boc-Gly, Boc-Thr(Bzl), Boc-Asp(OBzl), Boc-Ala, Boc-Ala, Boc-Ala, Boc-Gln, Boc-Gln and Boc-Ala.

The completed resin,

Boc-Ala-Gln-Gln-Ala-Ala-Ala-

Asp(OBzl)-Thr(Bzl)-Gly-His(DNP)-Ser(Bzl)-Ser(Bzl)-Gln-Val-

Ser(Bzl)-Gln-Asn-Tyr(BrZ)-$OCH_2$-$C_6H_4$-Resin

thus obtained weighed 1.95 g. The protected peptide resin was first treated with thiophenol in DMF for 1 hour to remove DNP-group from the histidine residue of the peptide and then stirred with anhydrous HF at 0° for 45 min for cleavage and deprotection. Work up the reaction afforded 360 mg of crude peptide. Part of the crude product was purified on the HPLC giving a material which was largely homogeneous an analytical HPLC (with a small slower moving minor contaminant).

Amino Acid Anal. Asp, 2.10; Thr, 1.01; Ser, 2.89; Glu, 3.91; Gly, 1.07; Ala, 4.05; Val, 1.09; Thr, 1.07; His, 0.81.

Example 4 - Preparation of Peptide C (56-Ser, 85-Lys)

Glu-Thr-Ser-Glu-Gly-Ser-Arg-Gln-Ile-Leu-Gly-Gln-Leu-

Gln-Pro-Ser-Leu-Gln-Thr-Gly-Ser-Glu-Glu-Leu-Arg-Ser-

Leu-Tyr-Asn-Thr-Val-Ala-Thr-Leu-Lys

Boc-Lys(ClZ)-$OCH_2$-$C_6H_4$-Resin (0.7 g; 0.35 mmol) was placed in a peptide synthesis flask and the cycles of the solid phase peptide synthesis carried out according to the schedule described above in Example 1 with the following Boc-amino acids (1.1 mmol each) sequentially coupled to the peptide chain on the resin, one at a time:

Boc-Leu, Boc-Thr(Bzl), Boc-Ala, Boc-Val, Boc-Thr(Bzl), Boc-Asn, Boc-Tyr(BrZ), Boc-Leu, Boc-Ser(Bzl), Boc-Arg(Tos), Boc-Leu, Boc-Glu(OBzl), Boc-Glu(OBzl), Boc-Ser(Bzl), Boc-Gly, Boc-Thr(Bzl), Boc-Gln, Boc-Leu, Boc-Ser(Bzl), Boc-Pro, Boc-Gln, Boc-Leu, Boc-Gln, Boc-Gly, Boc-Leu, Boc-Ile, Boc-Gln, Boc-Arg(Tos), Boc-Ser(Bzl;), Boc-Gly, Boc-Glu(OBzl), Boc-Ser(Bzl), Boc-Thr(Bzl) and Boc-Glu(OBzl).

The completed resin,

Boc-Glu(OBzl)-Thr(Bzl)-Ser(Bzl)-Glu(OBzl)-Gly-Ser(Bzl)-Arg(Tos)-Gln-Ile-Leu-Gly-Gln-Leu-Gln-Pro-Ser(Bzl)-Leu-Gln-Thr(Bzl)-Gly-Ser(Bzl)-Glu(OBzl)-Glu(OBzl)-Leu-Arg(Tos)-Ser(Bzl)-Leu-Tyr(BrZ)-Asn-Thr(Bzl)-Val-Ala-Thr(Bzl)-Leu-Lys(ClZ)-$OCH_2$-$C_6H_4$-Resin,

thus obtained weighed 1.28 g. HF cleavage and deprotection gave 457 mg of crude peptide. Purification on HPLC afforded 31 mg of desired peptide. The product was found to be homogeneous by analytical HPLC.

Amino Acid Anal. Asp, 1.00; Thr, 4.15; Ser, 4.32; Glu, 7.88; Pro, 0.76; Gly, 3.02; Ala, 0.90; Val, 0.95; Ile, 0.85; Leu, 5.99; Tyr, 0.97; Lys, 0:98; Arg, 1.95.

Example 5 - Preparation of Peptide A (33-Lys)

Gly-Ala-Arg-Ala-Ser-Val-Leu-Ser-Gly-Gly-Glu-Leu-Asp-Arg-Trp-Glu-Lys-Ile-Arg-Leu-Arg-Pro-Gly-Gly-Lys-Lys-Lys-Tyr-Lys- Leu-Lys-His-Lys

Boc-Lys(ClZ)-PAM-Resin (1.0 g; 0.32 mmol) was placed in the peptide synthesis flask and the cycles of the solid phase peptide synthesis carried out according to the schedule described above in Example 1 with the following Boc-amino acids (1.0 mmol each) sequentially coupled to the growing peptide chain on the resin, one amino acid derivative at a time:

Boc-His(DNP), Boc-Lys(ClZ), Boc-Leu, Boc-Lys(ClZ), Boc-Tyr(BrZ), Boc-Lys(ClZ), Boc-Lys(ClZ), Boc-Lys(ClZ), Boc-Gly, Boc-Gly, Boc-Pro, Boc-Arg(Tos), Boc-Leu, Boc-Arg(Tos), Boc-Ile, Boc-Lys(ClZ), Boc-Glu-(OBzl), Boc-Trp(For), Boc-Arg(Tos), Boc-Asp(OBzl), Boc-Leu, Boc-Glu(OBzl), Boc-Gly, Boc-Gly, Boc-Ser-(Bzl), Boc-Leu, Boc-Val, Boc-Ser(Bzl), Boc-Ala, Boc-Arg(Tos), Boc-Ala and Boc-Gly.

The completed resin,

Boc-Gly-Ala-Arg(Tos)-Ala-Ser(Bzl)-Val-Leu-Ser(Bzl)-Gly-Gly-Glu(OBzl)-Leu-Asp(OBzl)-Arg(Tos)-Trp(For)-Glu(OBzl)-Lys(ClZ)-Ile-Arg(Tos)-Leu-Arg(Tos)-Pro-Gly-Gly-Lys(ClZ)-Lys(ClZ)-Lys(ClZ)-Tyr(BrZ)-Lys(ClZ)-Leu-Lys(ClZ)-His(DNP)-Lys(ClZ)-PAM-Resin,

thus obtained weighed 2.23 g. The peptide-resin was treated with thiophenol in DMF for 1 hour to remove the DNP protecting group from the histidine residue on the peptide and then treated with anhydrous HF (0°, 60 min) in the presence of anisole, dimethyl sulfide, m-cresol and thiocresol. After removal of the excess HF, and dimethyl sulfide, fresh HF was introduced and the mixture was allowed to react at 0° for 45 min. Work up as usual yielded 580 mg of crude peptide. Analytical HPLC indicated that the material was about 90% pure. It was used without further attempt for purification.

Amino Acid Anal. Asp, 1.00; Ser, 1.45; Glu, 1.82; Pro, 0.61; Gly, 5.11; Ala, 1.73; Val, 0.85; Ile, 0.74; Leu, 3.73; Tyr, 0.89; Trp, (acid hydr., not determined); Lys, 6.22; His, 0.58; Arg, 3.52.

## Claims

1. A peptide having specific immunoreactivity with antibodies to HIV p17 selected from the group consisting of a peptide containing from about 28 to about 42 amino acids, the sequence of which corresponds to a part or the entirety of the following sequence:

   Glu-Thr-Ser-Glu-Gly-Cys-Arg-Gln-Ile-Leu-
   Gly-Gln-Leu-Gln-Pro-Ser-Leu-Gln-Thr-Gly-
   Ser-Glu-Glu-Leu-Arg-Ser-Leu-Tyr-Asn-Thr-
   Val-Ala-Thr-Leu (IV);

   analogues thereof wherein the amino acids in the sequence may be substituted, deleted or added, so long as the immunoreactivity to antibodies to HIV p17 derived from the three dimensional conformation of the sequence is preserved; and conjugates of the peptides or analogues thereof.

2. A peptide according to claim 1 containing the thirty-four amino acid sequence of formula (IV).

3. A peptide according to claim 2 having the formula:

   Glu-Thr-Ser-Glu-Gly-Ser-Arg-Gln-Ile-Leu-
   Gly-Gln-Leu-Gln-Pro-Ser-Leu-Gln-Thr-Gly-
   Ser-Glu-Glu-Leu-Arg-Ser-Leu-Tyr-Asn-Thr-
   Val-Ala-Thr-Leu-Lys.

4. A mixture of two or more peptides exhibiting specific immunoreactivity with antibodies to HIV p17 selected from the group consisting of (1) a peptide containing from about 10 to about 36 amino acids, the sequence of which corresponds to a part or the entirety of the sequence of formula (I'):

   Ile-$X_1$-$X_2$-Lys-Asp-Thr-Lys-Glu-Ala-Leu-
   $X_3$-Lys-Ile-Glu-Glu-Glu-Gln-Asn (I')

   wherein $X_1$ is Asp or Glu, $X_2$ is Val or Ile and $X_3$ is Glu or Asp;

(2) a peptide containing from about 26 to about 40 amino acids, the sequence of which corresponds to a part or the entirety of the sequence of formula (II):

Gly-Ala-Arg-Ala-Ser-Val-Leu-Ser-Gly-Gly-
Glu-Leu-Asp-Arg-Trp-Glu-Lys-Ile-Arg-Leu-
Arg-Pro-Gly-Gly-Lys-Lys-Lys-Tyr-Lys-Leu-
Lys-His-Ile (II);

(3) a peptide containing from about 14 to about 27 amino acids, the sequence of which corresponds to a part or the entirety of the sequence of formula (III):

Val-Trp-Ala-Ser-Arg-Glu-Leu-Glu-Arg-Phe-
Ala-Val-Asn-Pro-Gly-Leu-Leu-Glu-Thr (III);

(4) a peptide containing from about 28 to about 42 amino acids, the sequence of which corresponds to a part or the entirety of the sequence of formula (IV):

Leu-Glu-Thr-Ser-Glu-Gly-Cys-Arg-Gln-Ile-
Leu-Gly-Gln-Leu-Gln-Pro-Ser-Leu-Gln-Thr-
Gly-Ser-Glu-Glu-Leu-Arg-Ser-Leu-Tyr-Asn-
Thr-Val-Ala-Thr-Leu (IV);

(5) analogues of these peptides wherein the amino acids in said sequences may be substituted, deleted, or added, so long as the immunoreactivity to antibodies to HIV p17 derived from the three dimensional conformation of the sequence is preserved; and
(6) conjugates of said peptides and analogues thereof, wherein at least one of the peptides is of formula (IV) or is a said analogue thereof or a conjugate or analogue thereof.

**5.** An immunogenic antigen comprising the peptide of any one of claims 1 to 3 covalently bonded to an immunogenic carrier material.

**6.** An immunogenic antigen according to claim 5 wherein said peptide is labelled for the serological detection of an antibody to said antigen.

**7.** A vaccine for use in the prevention or treatment of Acquired Immune Deficiency Syndrome (AIDS) or AIDS-Related Complex (ARC) which comprises an amount in the range of from about 50 to 200 μg of the antigenic peptide of any one of claims 1 to 3 or a mixture of antigenic peptides according to claim 4, and a pharmaceutically effective carrier.

**8.** A method for detection of AIDS virus in body fluids of a human patient which comprises contacting at least one sample of the body fluid with one or more antibodies to the peptides of any one of claims 1 to 3 or to the mixture of peptides of claim 4, and measuring the formation of antigen-antibody complexes by radioimmunoassay, enzyme linked immunosorbent assay or indirect immunofluorescent assay.

**9.** The method of claim 8 which comprises contacting a sample of body fluid with the mixture of the peptides according to claim 4, and thereafter measuring the formation of antigen-antibody complex for each peptide in said mixture.

**10.** The method of claim 8 which comprises contacting a plurality of samples of body fluid from said patient with a single different antigenic peptide for each sample, and thereafter, measuring the formation of antigen-antibody complex in each sample.

**11.** A diagnostic test kit for detection of antibodies recognizing epitopes of p17 protein of HIV in human body fluid comprising a compartmented enclosure containing multiwell plates which are coated with one or more peptides according to any one of claims 1 to 3 or the mixture of peptides according to claim 4, and ELISA materials for enzyme detection including normal animal antisera and enzyme, labelled anti-antibody antisera and a color change indicator.

**12.** A diagnostic kit according to claim 11 which comprises a plurality of multiwell plates, the wells of each plate being coated with a different one of said peptides.

**13.** A diagnostic kit according to claim 11 wherein the wells of said multiwell plates are coated with a mixture of two or more of said peptides.

**14.** A method for forming an antibody enriched sera for use in treatment of AIDS which comprises withdrawing a sample of body fluid from an individual testing positive for anti-p17 antibodies to HIV but which individual does not exhibit any symptoms of AIDS or ARC, determining the presence of antibodies to the immunodominant epitopes of one or more of the peptides selected from the group consisting of (1) a peptide containing from about 10 to about 36 amino acids, the sequence of which corresponds to a part or the entirety of the sequence of formula (I'):

Ile-$X_1$-$X_2$-Lys-Asp-Thr-Lys-Glu-Ala-Leu-
$X_3$-Lys-Ile-Glu-Glu-Glu-Gln-Asn (I')

wherein $X_1$ is Asp or Glu, $X_2$ is Val or Ile, and $X_3$ is Glu or Asp;
(2) a peptide containing from about 26 to about 40 amino acids, the sequence of which corresponds to a part or the entirety of the sequence of formula (II):

Gly-Ala-Arg-Ala-Ser-Val-Leu-Ser-Gly-Gly-
Glu-Leu-Asp-Arg-Trp-Glu-Lys-Ile-Arg-Leu-
Arg-Pro-Gly-Gly-Lys-Lys-Lys-Tyr-Lys-Leu-
Lys-His (II);

(3) a peptide containing from about 14 to about 27 amino acids, the sequence of which corresponds to a part or the entirety of the sequence of formula (III):

Ile-Val-Trp-Ala-Ser-Arg-Glu-Leu-Glu-Arg-
Phe-Ala-Val-Asn-Pro-Gly-Leu-Leu (III);

(4) a peptide containing from about 28 to about 42 amino acids, the sequence of which corresponds to a part or the entirety of the sequence of formula (IV):

Glu-Thr-Ser-Glu-Gly-Cys-Arg-Gln-Ile-Leu-
Gly-Gln-Leu-Gln-Pro-Ser-Leu-Gln-Thr-Gly-
Ser-Glu-Glu-Leu-Arg-Ser-Leu-Tyr-Asn-Thr-
Val-Ala-Thr-Leu (IV);

(5) analogues of these peptides wherein the amino acids in said sequences may be substituted, deleted, or added, so long as the immunoreactivity to antibodies to HIV p17 derived from the three dimensional conformation of the sequence is preserved; and
(6) conjugates of said peptides and analogues thereof, wherein the or at least one of the peptides is of formula (IV) or is a said active analogue thereof or a conjugate or analogue thereof, and subjecting at least one sample of body fluid from said individual to affinity chromatography to remove any of said antibodies which do not confer protection against development of AIDS and/or to enrich the sera for any of said antibodies which do confer protection against development of AIDS.

FIG. 1

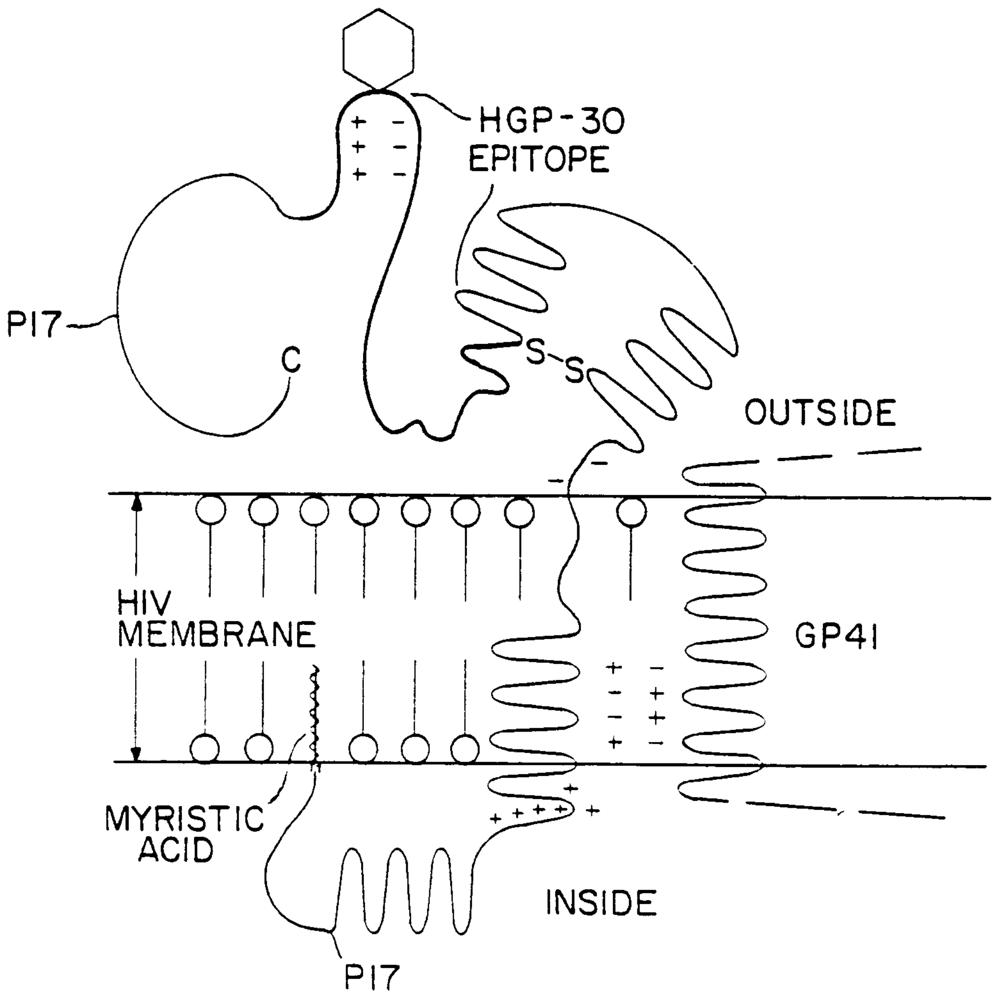
HGP-30
EPITOPE
P17
C
S-S
OUTSIDE
HIV
MEMBRANE
GP41
MYRISTIC
ACID
INSIDE
P17

## COMPARISON OF THE AMINO ACID SEQUENCE OF P17's

```
HIV-2               +   +   +  Asn  +   +   +  Arg  +  Lys Lys Ala  +  Glu
CH3(CH2)12CC-Gly Ala Arg Ala Ser Val Leu Ser Gly Gly Glu Leu Asp Arg
SIV                 +   +   +  Asn  +   +   +   +   +  Lys Lys Ala  +  Glu

Leu  +   +  Arg  +   +   +   +   +   +   +   +   +   +  Arg  +   +   +
Trp Glu Lys Ile Arg Leu Arg Pro Gly Gly Lys Lys Lys Tyr Lys Leu Lys His  HGP-33
Leu  +   +   +   +   +   +   +   +   +   +   +   +   +  Met  +   +   +

 +   +   +   +  Ala Asn Lys  +  Asp  +   +  Gly Leu Ala Ala Ser  +   +
Ile Val Trp Ala Ser Arg Glu Leu Glu Arg Phe Ala Val Asn Pro Gly Leu Leu  HGP-19
Val  +   +   +  Ala Asn  +   +  Asp  +   +  Gly Leu Ala Glu Ser  +   +

 +  Ser Lys  +   +   +  Gln Lys  +   +  Thr Val  +  Asp  +  Met Val Pro
Glu Thr Ser Glu Gly Cys Arg Gln Ile Leu Gly Gln Leu Gln Pro Ser Leu Gln  HGP-35
 +  Asn Lys  +   +   +  Gln Lys  +   +  Ser Val  +  Ala  +  Leu Val Pro
```

FIG. 2-1

\+ + + + Asn + Lys + + Phe + + + Cys Val Ile Trp +
Thr Gly Ser Glu Glu Leu Arg Ser Leu Tyr Asn Thr Val Ala Thr Leu Tyr Cys
\+ + + + Asn + Lys + + + + + + Cys Val Ile Trp Cys

Ile + Ala Glu Gln Lys Val + + + Glu Gly + Lys Gln Ile Val Arg
Val His Gln Arg Ile Glu Ile Lys Asp Thr Lys Glu Ala Leu Asp Lys Ile Glu HGP-30
Ile + Ala Glu Glu Lys Val + His + Glu + + Lys Gln Ile Val Gln

Glu Ala Glu Asn
Arg His Leu Val Ala Glu Thr Gly Thr + Glu Lys Met Pro Ser Thr Ser Arg
Glu Glu Gln Asn Lys Ser Lys Lys Lys Ala Gln Gln Ala Ala Ala Asp Thr Gly HGP-17
Arg His Leu Val Met Glu Thr Gly Thr + Glu Thr Met Pro Lys Thr Ser Arg
\+ + + +

Pro Thr Ala Pro Ser + Glu Lys Gly Gly Asn Tyr
His Ser Ser Gln Val Ser Gln Asn Tyr
Pro Thr Ala Pro Phe + Gly Arg Gly Gly Asn Tyr

FIG. 2-2

**PRIMARY AMINO ACID SEQUENCE of p17 Gag**

|←Myristic Acid→|←HGP-32

$CH_3(CH_2)_{12}CO$-Gly Ala Arg Ala Ser Val Leu Ser Gly Gly Glu

Leu Asp Arg Trp Glu Lys Ile Arg Leu Arg Pro Gly Gly Lys

→|←

Lys Lys Tyr Lys Leu Lys His Ile Val Trp Ala Ser Arg Glu

HGP-18 →|←

Leu Glu Arg Phe Ala Val Asn Pro Gly Leu Leu Glu Thr Ser

HGP-34

Glu Gly Cys Arg Gln Ile Leu Gly Gln Leu Gln Pro Ser Leu

Gln Thr Gly Ser Glu Glu Leu Arg Ser Leu Tyr Asn Thr Val

→| |←HGP-30

Ala Thr Leu Tyr Cys Val His Gln Arg Ile Glu Ile Lys Asp

Thr Lys Glu Ala Leu Asp Lys Ile Glu Glu Glu Gln Asn Lys

→|

|←Ala-HGP-17

Ser Lys Lys Lys Ala Gln Gln Ala Ala Ala Asp Thr Gly His

→|

Ser Ser Gln Val Ser Gln Asn Tyr-COOH

FIG. 3

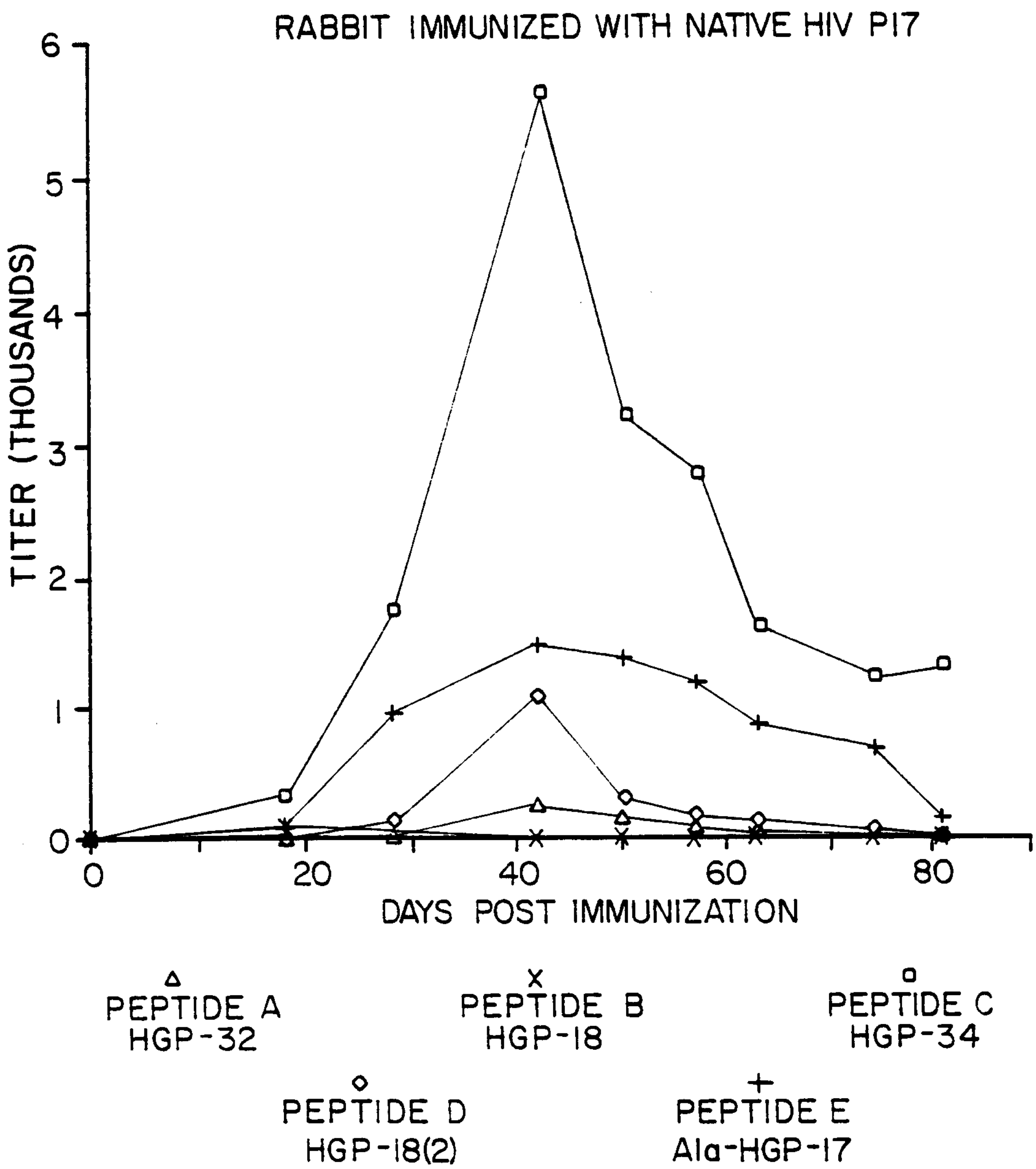
RABBIT IMMUNIZED WITH NATIVE HIV P17
TITER (THOUSANDS)
0
1
2
3
4
5
6
0
20
40
60
80
DAYS POST IMMUNIZATION
Δ PEPTIDE A HGP-32
x PEPTIDE B HGP-18
□ PEPTIDE C HGP-34
◇ PEPTIDE D HGP-18(2)
+ PEPTIDE E Ala-HGP-17

FIG. 4

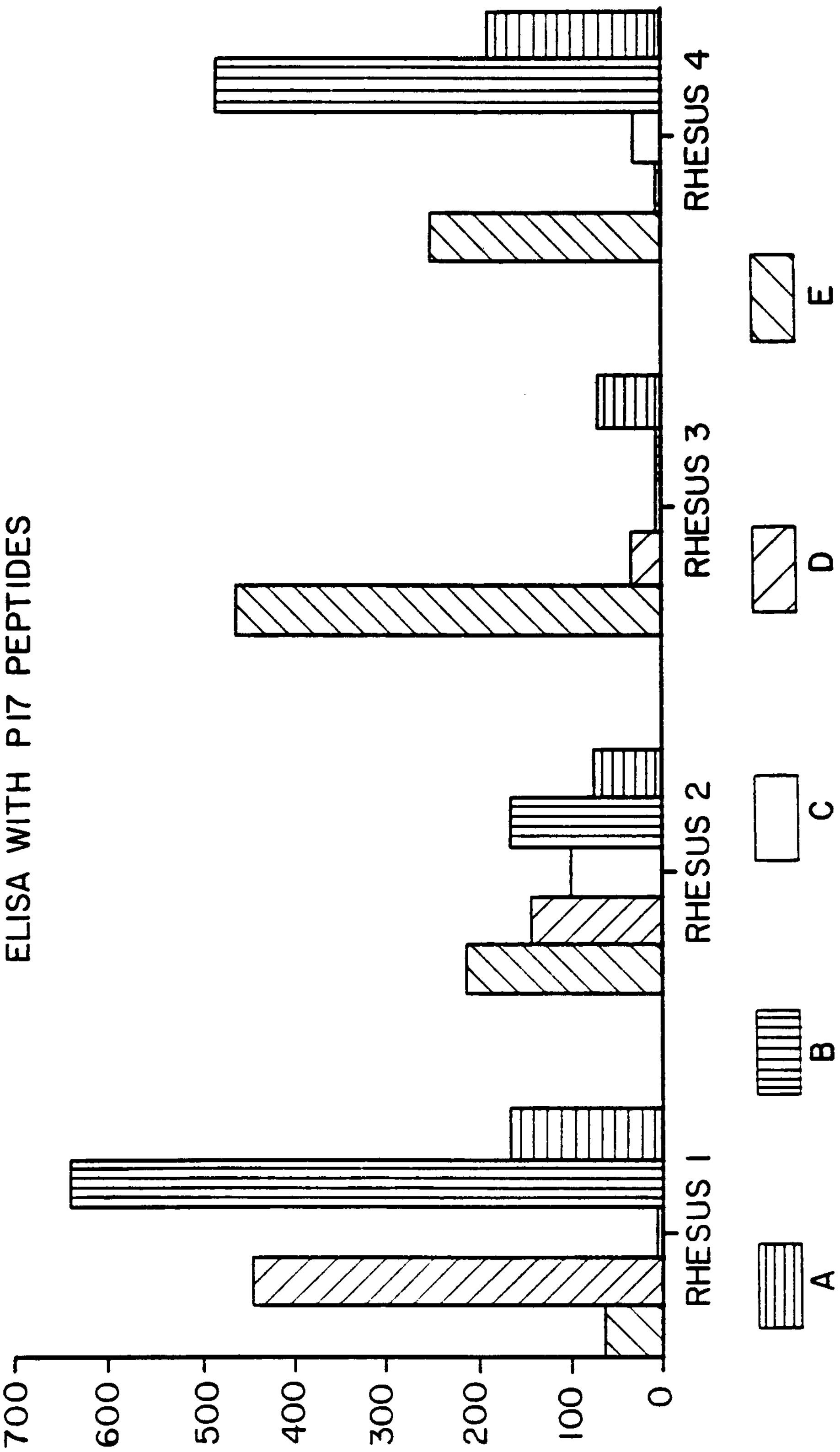
ELISA WITH P17 PEPTIDES
700
600
500
400
300
200
100
0
RHESUS 1
RHESUS 2
RHESUS 3
RHESUS 4
A
B
C
D
E

FIG. 5

European Patent Office

# EUROPEAN SEARCH REPORT

Application Number
EP 94 10 6294

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 284 587 (VIROVAHL S.A.) 28 September 1988<br>* page 5, line 40 - line 43 * | 1,2,5,6 | A61K39/21<br>C07K7/10<br>G01N33/569 |
| X,D | EP-A-0 230 222 (F.HOFFMANN-LAROCHE & CO) 29 July 1987<br>* figure 14 * | 1,2,5,6 | |
| X | EP-A-0 322 394 (SMITHKLINE BIOLOGICALS S.A.) 28 June 1989<br>* claim 8 * | 1,2,5,6 | |

TECHNICAL FIELDS SEARCHED (Int.Cl.5)

A61K
C07K
G01N

The present search report has been drawn up for all claims

| Place of search | Date of completion of the search | Examiner |
|---|---|---|
| THE HAGUE | 24 June 1994 | Cupido, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

SEQUENCE LISTING

(1) GENERAL INFORMATION:

(i) APPLICANT:
(A) NAME: VIRAL TECHNOLOGIES, INC.
(B) STREET: Two Democracy Center, 6903 Rockledge Drive, Suite 1200
(C) CITY: Bethesda
(D) STATE: Maryland
(E) COUNTRY: U.S.A.
(F) POSTAL CODE (ZIP): 20817

(A) NAME: THE GEORGE WASHINGTON UNIVERSITY
(B) STREET: Office of Sponsored Research, Rice Hall, 2121 Eye Street N.W
(C) CITY: Washington
(D) STATE: D.C.
(E) COUNTRY: U.S.A.
(F) POSTAL CODE (ZIP): 20052

(ii) TITLE OF INVENTION: HIV related peptides

(iii) NUMBER OF SEQUENCES: 11

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(vi) PRIOR APPLICATION DATA:
(A) APPLICATION NUMBER: US 420469
(B) FILING DATE: 12-OCT-1989

(vi) PRIOR APPLICATION DATA:
(A) APPLICATION NUMBER: EP 90311056.7
(B) FILING DATE: 09-OCT-1990

(2) INFORMATION FOR SEQ ID NO: 1:

(i) SEQUENCE CHARACTERISTICS:
(A) LENGTH: 30 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 1:

```
Tyr Ser Val His Gln Arg Ile Asp Val Lys Asp Thr Lys Glu Ala Leu
1               5                   10                  15

Glu Lys Ile Glu Glu Glu Gln Asn Lys Ser Lys Lys Lys Ala
            20                  25                  30
```

(2) INFORMATION FOR SEQ ID NO: 2:

(i) SEQUENCE CHARACTERISTICS:
(A) LENGTH: 32 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 2:

```
Gly Ala Arg Ala Ser Val Leu Ser Gly Gly Glu Leu Asp Arg Trp Glu
1               5                   10                  15

Lys Ile Arg Leu Arg Pro Gly Gly Lys Lys Lys Tyr Lys Leu Lys His
            20                  25                  30
```

(2) INFORMATION FOR SEQ ID NO: 3:

(i) SEQUENCE CHARACTERISTICS:
(A) LENGTH: 18 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 3:

```
Ile Val Trp Ala Ser Arg Glu Leu Glu Arg Phe Ala Val Asn Pro Gly
1               5                   10                  15

Leu Leu
```

(2) INFORMATION FOR SEQ ID NO: 4:

(i) SEQUENCE CHARACTERISTICS:
(A) LENGTH: 34 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 4:

```
Glu Thr Ser Glu Gly Cys Arg Gln Ile Leu Gly Gln Leu Gln Pro Ser
1               5                   10                  15

Leu Gln Thr Gly Ser Glu Glu Leu Arg Ser Leu Tyr Asn Thr Val Ala
```

20 25 30

Thr Leu

(2) INFORMATION FOR SEQ ID NO: 5:

(i) SEQUENCE CHARACTERISTICS:
(A) LENGTH: 18 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 5:

Ala Gln Gln Ala Ala Ala Asp Thr Gly His Ser Ser Gln Val Ser Gln
1 5 10 15

Asn Tyr

(2) INFORMATION FOR SEQ ID NO: 6:

(i) SEQUENCE CHARACTERISTICS:
(A) LENGTH: 18 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(ix) FEATURE:
(A) NAME/KEY: Modified-site
(B) LOCATION: 2
(D) OTHER INFORMATION: /note= "Xaa is Glu or Asp"

(ix) FEATURE:
(A) NAME/KEY: Modified-site
(B) LOCATION: 3
(D) OTHER INFORMATION: /note= "Xaa is Ile or Val"

(ix) FEATURE:
(A) NAME/KEY: Modified-site
(B) LOCATION: 11
(D) OTHER INFORMATION: /note= "Xaa is Asp or Glu"

(xi) SEQUENCE DESCRIPTION: SEQ ID NO: 6:

Ile Xaa Xaa Lys Asp Thr Lys Glu Ala Leu Xaa Lys Ile Glu Glu Glu
1 5 10 15

Gln Asn

(2) INFORMATION FOR SEQ ID NO: 7:

(i) SEQUENCE CHARACTERISTICS:
(A) LENGTH: 18 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 7:

```
Ile Asp Val Lys Asp Thr Lys Glu Ala Leu Glu Lys Ile Glu Glu Glu
1               5                   10                  15

Gln Asn
```

(2) INFORMATION FOR SEQ ID NO: 8:

(i) SEQUENCE CHARACTERISTICS:
(A) LENGTH: 19 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 8:

```
Ile Val Trp Ala Ser Arg Glu Leu Glu Arg Phe Ala Val Asn Pro Gly
1               5                   10                  15

Leu Leu Tyr
```

(2) INFORMATION FOR SEQ ID NO: 9:

(i) SEQUENCE CHARACTERISTICS:
(A) LENGTH: 35 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 9:

```
Glu Thr Ser Glu Gly Ser Arg Gln Ile Leu Gly Gln Leu Gln Pro Ser
1               5                   10                  15
```

```
Leu Gln Thr Gly Ser Glu Glu Leu Arg Ser Leu Tyr Asn Thr Val Ala
            20                  25                  30

Thr Leu Lys
        35
```

(2) INFORMATION FOR SEQ ID NO: 10:

(i) SEQUENCE CHARACTERISTICS:
(A) LENGTH: 33 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 10:

```
Gly Ala Arg Ala Ser Val Leu Ser Gly Gly Glu Leu Asp Arg Trp Glu
1               5                   10                  15

Lys Ile Arg Leu Arg Pro Gly Gly Lys Lys Lys Tyr Lys Leu Lys His
            20                  25                  30

Lys
```

(2) INFORMATION FOR SEQ ID NO: 11:

(i) SEQUENCE CHARACTERISTICS:
(A) LENGTH: 131 amino acids
(B) TYPE: amino acid
(D) TOPOLOGY: unknown

(ii) MOLECULE TYPE: protein

(vi) ORIGINAL SOURCE:
(A) ORGANISM: p17 gag protein of HIV-1 virus
(B) STRAIN: SF-2

(xi) SEQUENCE DESCRIPTION: SEQ ID NO: 11:

```
Gly Ala Arg Ala Ser Val Leu Ser Gly Gly Glu Leu Asp Arg Trp Glu
1               5                   10                  15

Lys Ile Arg Leu Arg Pro Gly Gly Lys Lys Lys Tyr Lys Leu Lys His
            20                  25                  30

Ile Val Trp Ala Ser Arg Glu Leu Glu Arg Phe Ala Val Asn Pro Gly
        35                  40                  45

Leu Leu Glu Thr Ser Glu Gly Cys Arg Gln Ile Leu Gly Gln Leu Gln
    50                  55                  60

Pro Ser Leu Gln Thr Gly Ser Glu Glu Leu Arg Ser Leu Tyr Asn Thr
65                  70                  75                  80

Val Ala Thr Leu Tyr Cys Val His Gln Arg Ile Glu Ile Lys Asp Thr
                85                  90                  95
```

```
Lys Glu Ala Leu Asp Lys Ile Glu Glu Glu Gln Asn Lys Ser Lys Lys
            100                 105                 110

Lys Ala Gln Gln Ala Ala Ala Asp Thr Gly His Ser Ser Gln Val Ser
        115                 120                 125

Gln Asn Tyr
    130
```